# EUROPÄISCHE PATENTSCHRIFT

(11) **EP 2 509 574 B1**
(45) Veröffentlichungstag und Bekanntmachung des Hinweises auf die Patenterteilung: **18.01.2017**
(21) Anmeldenummer: 10778659.2
(22) Anmeldetag: 17.11.2010
(51) Int. Cl.: A61Q 5/06, A61K 8/895, A61K 8/81

(54) **POLYMERKOMBINATION FÜR GLANZGEBENDE KOSMETISCHE HAARBEHANDLUNGSMITTEL MIT STARKEM HALT UND GUTEM HAARGEFÜHL**
POLYMER COMBINATION FOR A SHINE-PRODUCING COSMETIC HAIR TREATMENT AGENT HAVING STRONG HOLD AND GOOD HAIR FEEL
COMBINAISON DE POLYMÈRES POUR DES AGENTS COSMÉTIQUES DE TRAITEMENT CAPILLAIRE DONNANT DE LA BRILLANCE, PERMETTANT D'OBTENIR UN MAINTIEN FERME ET UN BON TOUCHER DES CHEVEUX

(30) Priorität: 10.12.2009 DE 102009054516
(43) Veröffentlichungstag der Anmeldung: 17.10.2012
(73) Patentinhaber: Henkel AG & Co. KGaA, 40589 Düsseldorf (DE)
(72) Erfinder: SCHWEINSBERG, Matthias, 22769 Hamburg (DE); BAUMSCHEIPER, Michael, 25436 Heidgraben (DE); MÜLLER, Burkhard, 21075 Hamburg (DE)
(86) Internationale Anmeldenummer: PCT/EP2010/067621
(87) Internationale Veröffentlichungsnummer: WO 2011/069786

(56) Entgegenhaltungen:
- EP-A2- 2 106 784
- SCHWARZWAELDER CLAUDIUS ET AL: "New hybrid polymer for hair spray formulations", COSMETICS & TOILETRIES, WHEATON, IL, US, Bd. 122, Nr. 12, 3. Dezember 2007 (2007-12-03), Seiten 53-54,56,58, XP009168302, ISSN: 0361-4387
- "Dimethicone Crosspolymers Ingredients as Used in Cosmetics", , 16. Februar 2012 (2012-02-16), Seiten 1-49, XP055057785, Gefunden im Internet: URL:http://www.cir-safety.org/sites/defaul t/files/Dimeth032012SLR_forposting.pdf [gefunden am 2013-03-26]

## Beschreibung

Die vorliegende Erfindung betrifft Mittel zur Haarbehandlung, enthaltend mindestens ein spezielles anionisches Copolymer abgeleitet von vinylaromatischen Verbindungen in Kombination mit mindestens einem speziellen anionischem Copolymer mit Silikongruppen, die Verwendung dieser Mittel zur temporären Verformung und/oder zur Pflege keratinhaltiger Fasern und Aerosolsprays bzw. Aerosolschäume auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die zunächst wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bereits bei geringer Einsatzmenge bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Außerdem fühlt sich das mit einem festigenden Polymer beschichtete Haar oftmals rauh an.

Weiterhin soll das temporär gestylte Haar neben dem starken Frisurenhalt gesund und natürlich aussehen. Dabei spielt der Haarglanz eine herausragende Rolle. Oftmals werden deshalb den Haarstylingmitteln sogenannte Glanzbildner in ausreichender Menge zugesetzt. Diese Glanzbildner sind beispielsweise Öle oder glanzgebende Pigmente wie z.B. Glimmerpartikel. Glanzgebende Partikel weisen den Nachteil auf, dass sie sich mit der Zeit vom Haar lösen und sich nach einiger Zeit z.B. auf der Kleidung oder der Gesichtshaut wieder finden. Öle beschweren das Haar und führen teilweise zu einer verschlechterten Haftung der filmbildenden bzw. festigenden Polymere auf dem Haar. Dies führt möglicherweise zu dem Nachteil, dass die aufgeprägte Frisur durch die filmbildenden bzw. festigenden Polymere nicht über eine ausreichende Dauer fixiert werden kann. Die Frisur hängt sich schneller aus.

Die europäische Patentanmeldung EP 2 106 784 A1 betrifft Haarstylingzusammensetzungen, welche mindestens ein lösliches Polymer auf Basis von Acrylat- und Styrolmonomeren sowie ein Lösungsmittelgemisch oder ein Treibmittelgemisch enthalten.

Weiterhin betrifft die wissenschaftliche Publikation von Schwarzwälder et. al. "New Hybrid Polymer for hair spray formulations", Cosmetics & Toiletries' magazine, Vol. 122, 2007, Seiten 53 bis 60 Siliconpolymere auf Basis von Divinylpolydimethylsiloxanen für die Anwendung in Haarsprayformulierungen.

Aufgabe der vorliegenden Erfindung war es daher, ein Mittel zur temporären Verformung und/oder zur Pflege keratinischer Fasern zur Verfügung zu stellen, das sich durch einen hohen Haltegrad auszeichnet, einen hervorragenden Glanz auf den keratinhaltigen Fasern und eine gute Sensorik der keratinhaltigen Fasern (insbesondere ein weiches Haargefühl) bewirkt.

Es wurde nunmehr überraschenderweise gefunden, dass dies durch eine Kombination eines speziellen festigenden anionischen Copolymers mit einem weiteren speziellen vernetzten anionischen Copolymer erreicht werden kann.

Ein erster Gegenstand der vorliegenden Erfindung ist daher ein Mittel zur Behandlung keratinhaltiger Fasern, insbesondere menschlichem Haar, enthaltend in einem kosmetisch akzeptablen Träger
- mindestens ein erstes festigendes anionisches Copolymer, umfassend mindestens eine Struktureinheit der Formel (I) und mindestens eine Struktureinheit der Formel (II) und mindestens eine Struktureinheit der Formel (III) 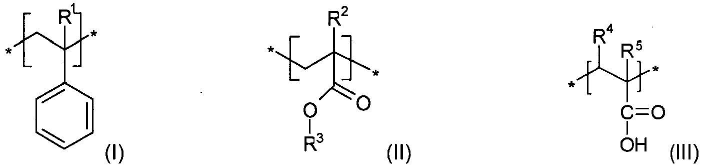 worin
   - R¹: steht für ein Wasserstoffatom oder eine Methylgruppe,
   - R²: steht für ein Wasserstoffatom oder eine Methylgruppe
   - R³: steht für eine (C₁ bis C₂₀)-Alkylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
   - R⁴ und R⁵: stehen für ein Wasserstoffatom oder einer der beiden Reste für ein Wasserstoffatom und der andere für eine Methylgruppe
   und
- mindestens ein zweites vernetztes anionisches Copolymer umfassend mindestens eine vernetzende Polysiloxan-Struktureinheit der Formel (PS) und mindestens eine Struktureinheit der Formel (IV) und mindestens eine Struktureinheit der Formel (V) 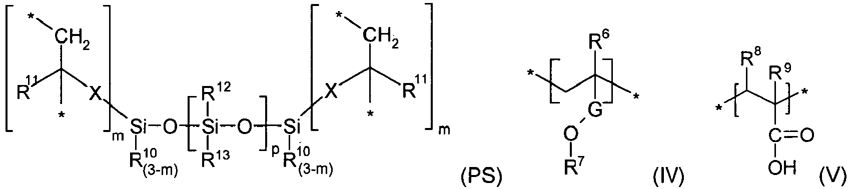 worin
   - R⁶: ein Wasserstoffatom oder eine Methylgruppe bedeutet,
   - R⁷: steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
   - G: eine direkte Bindung oder eine Carbonylgruppe bedeutet,
   - R⁸ und R⁹: stehen für ein Wasserstoffatom oder einer der beiden Reste für ein Wasserstoffatom und der andere für eine Methylgruppe
   R¹¹ steht für ein Wasserstoffatom oder eine Methylgruppe,
   R¹⁰, R¹², R¹³ stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
   m für eine ganze Zahl von 1 bis 3 steht
   p für eine ganze Zahl von 10 bis 1000 steht,
   X steht für eine direkte Bindung oder ein beliebiges Molekülfragment.

Gemäß obiger Formeln und allen folgenden Formeln steht eine chemische Bindung, die mit dem Symbol * gekennzeichnet ist, für eine "freie Valenz" des entsprechenden Strukturfragments. An diese "freie Valenz" binden im entsprechenden Copolymer weitere Strukturfragmente. Dies sind zuvor beschriebene und gegebenenfalls zusätzlich weitere Strukturfragmente des jeweiligen Polymers.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden. Als eine Testmethode für die festigende Wirkung eines Polymers wird häufig der so genannte curl-retention - Test angewendet.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Unter "vernetzt" bzw. "Vernetzung" ist im Sinne der Erfindung die Verknüpfung von Polymerketten miteinander durch kovalente chemische Bindung unter Bildung eines Netzwerkes zu verstehen. Diese kovalente Verknüpfung der Polymerketten erfolgt durch ein verbrückendes Polysiloxan-Molekülfragment. Das vernetzende Polysiloxan-Molekülfragment bindet an die durch das Molekülfragment verbrückten Polymerketten jeweils mittels kovalenter chemischer Bindung.

Unter einem anionischen Polymer wird erfindungsgemäß ein Polymer verstanden, das in einem protischen Lösemittel bei Standardbedingungen Struktureinheiten mit anionischen Gruppen, welche durch Gegenionen unter Erhaltung der Elektroneutralität kompensiert werden müssen, trägt und keine Struktureinheiten mit permanent kationischen oder kationisierbaren Gruppen aufweist. Unter anionische Gruppen fallen Carboxyl- und Sulfonsäuregruppen.

Das erfindungsgemäße Mittel enthält das erste festigende anionische Copolymer bevorzugt in einer Menge von 0.01 bis 29.99 Gew.-%, besonders bevorzugt von 0,1 bis 14,9 Gew.-%, ganz besonders bevorzugt von 0,1 bis 9,5 Gew.-%, und am bevorzugtesten von 0,2 bis 5,0 Gew.-%, jeweils bezogen auf das Gewicht des gesamten anwendungsbereiten Mittels.

Das erfindungsgemäße Mittel enthält das zweite vernetzte anionische Copolymer bevorzugt in einer Menge von 0.01 bis 29.99 Gew.-%, besonders bevorzugt von 0,1 bis 14,9 Gew.-%, ganz besonders bevorzugt von 0,1 bis 9,5 Gew.-%, und am bevorzugtesten von 0,2 bis 5,0 Gew.-%, jeweils bezogen auf das Gewicht des gesamten anwendungsbereiten Mittels.

Außerdem hat es sich als bevorzugt erwiesen, wenn das erfindungsgemäße Mittel eine totale Menge festigender Polymere von 0,01 bis 30 Gew.-%, besonders bevorzugt von 0,1 bis 15 Gew.-%, ganz besonders bevorzugt von 0,5 bis 10,0 Gew.-%, am bevorzugtesten von 1,0 bis 6,0 Gew.-%, jeweils bezogen auf das Gewicht des gesamten anwendungsbereiten Mittels.

Bevorzugt geeignete erste festigende anionische Copolymere, umfassend mindestens eine Struktureinheit der Formel (I) und mindestens eine Struktureinheit der Formel (IIa) und mindestens eine Struktureinheit der Formel (IIb) und mindestens eine Struktureinheit der Formel (III) 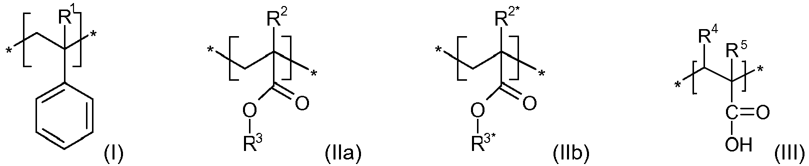 worin
R¹ steht für ein Wasserstoffatom oder eine Methylgruppe,
R² steht für ein Wasserstoffatom oder eine Methylgruppe
R³ steht für eine (C₁ bis C₂₀)-Alkylgruppe oder eine Gruppe *-(CH₂CH₂O)ₙ(C₁ bis C₂₀)-Alkyl mit n = 1 bis 30,
R² steht für ein Wasserstoffatom oder eine Methylgruppe
R^{3*} steht für eine (C₂ bis C₆)-Hydroxyalkylgruppe,
R⁴ und R⁵ stehen für ein Wasserstoffatom oder einer der beiden Reste für ein Wasserstoffatom und der andere für eine Methylgruppe.

Es stellten sich solche erfindungsgemäßen Mittel als besonders geeignet heraus, die für das erste festigende anionische Copolymer mindestens eines oder mehrere der folgenden Merkmale erfüllen:
- Bevorzugt steht der Rest R¹ gemäß Formel (I) für ein Wasserstoffatom.
- Bevorzugt steht R³ gemäß Formel (II) (bzw. R³ gemäß Formel (IIa) und R^{3*} gemäß Formel (IIb) unabhängig voneinander) für eine Methylgruppe, Ethylgruppe, 2-Hydroxyethylgruppe, 2,3-Dihydroxypropylgruppe, n-Propylgruppe, Isopropylgruppe, n-Butylgruppe, sec-Butylgruppe oder tert-Butylgruppe.
- Bevorzugt steht R³ gemäß Formel (IIa) für eine Methylgruppe, Ethylgruppe, n-Propylgruppe, Isopropylgruppe, n-Butylgruppe, sec-Butylgruppe oder tert-Butylgruppe.

- Bevorzugt steht R^{3*} gemäß Formel (IIb) für eine 2-Hydroxyethylgruppe oder 2,3-Dihydroxypropylgruppe.
- Bevorzugt steht R⁴ gemäß Formel (III) für ein Wasserstoffatom.

Bevorzugt sind daher solche erfindungsgemäßen Mittel, die in einem kosmetisch akzeptablen Träger neben dem besagten zweiten vernetzen anionischen Copolymer mindestens ein erstes festigendes anionisches Copolymer enthalten, umfassend mindestens eine Struktureinheit der Formel (I) und mindestens eine Struktureinheit der Formel (II) und mindestens eine Struktureinheit der Formel (III) 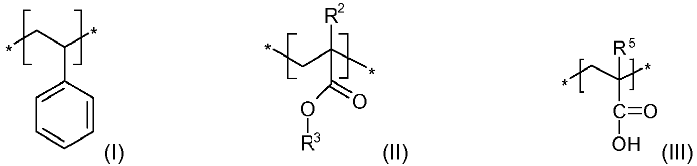 worin
R² steht für ein Wasserstoffatom oder eine Methylgruppe
R³ steht für eine (C₁ bis C₂₀)-Alkylgruppe, oder eine (C₂ bis C₆)-Hydroxyalkylgruppe, und
R⁵ steht für ein Wasserstoffatom oder eine Methylgruppe.

Ferner haben sich erfindungsgemäße Mittel als geeignet herausgestellt, in denen neben dem besagten zweiten vernetzten anionischen Copolymer mindestens ein erstes festigendes anionisches Copolymer enthalten ist, umfassend mindestens eine Struktureinheit der Formel (I) und mindestens eine Struktureinheit der Formel (IIa) und mindestens eine Struktureinheit der Formel (IIb) und mindestens eine Struktureinheit der Formel (III) 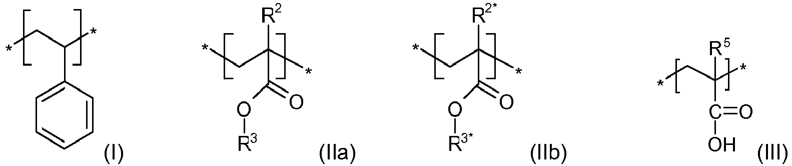 worin
R² steht für ein Wasserstoffatom oder eine Methylgruppe
R³ steht für eine (C₁ bis C₂₀)-Alkylgruppe,
R² steht für ein Wasserstoffatom oder eine Methylgruppe
R^{3*} steht für eine (C₂ bis C₄)-Hydroxyalkylgruppe (insbesondere 2-Hydroxyethyl oder 2,3-Dihydroxypropyl),
R⁵ steht für ein Wasserstoffatom oder eine Methylgruppe.

Die Menge der entsprechend im ersten festigenden anionischen Copolymer enthaltenen Struktureinheiten der Formel (I) entspricht bevorzugt 30 bis 60 Gew.-%, besonders bevorzugt 35 bis 55 Gew.-%, jeweils bezogen auf das Gewicht des besagten Polymers. Dies gilt für alle zuvor und später beschriebenen Ausführungsformen mutatis mutandis.

Die Menge der entsprechend im ersten festigenden anionischen Copolymer enthaltenen Struktureinheiten der Formel (II) (bzw. die Summe der Struktureinheiten der Formeln (IIa) und (IIb)) entspricht bevorzugt 1 bis 50 Gew.-%, besonders bevorzugt 5 bis 40 Gew.-%, ganz besonders bevorzugt von 10 bis 40 Gew.-%, jeweils bezogen auf das Gewicht des besagten Polymers. Dies gilt für alle zuvor und später beschriebenen Ausführungsformen mutatis mutandis.

Die Menge der entsprechend im ersten festigenden anionischen Copolymer enthaltenen Struktureinheiten der Formel (III) entspricht bevorzugt 5 bis 50 Gew.-%, besonders bevorzugt 10 bis 40 Gew.-%, jeweils bezogen auf das Gewicht des besagten Polymers. Dies gilt für alle zuvor und später beschriebenen Ausführungsformen mutatis mutandis.

Besonders bevorzugt handelt es sich bei dem ersten anionischen festigenden Copolymer um ein anionisches Copolymer, welches aus Monomeren umfassend Monomere (a) und (b) und (c) hergestellt wird:
(a) Styrol und/oder alpha-Methylstyrol (insbesondere Styrol),
(b) mindestens einem Monomer ausgewählt unter Methylacrylat, Ethylacrylat, 2-Hydroxyethylacrylat, n-Propylacrylat, Isopropylacrylat, n-Butylacrylat, tert.-Butylacrylat, Methylmethacrylat, Ethylmethacrylat, 2-Hydroxyethylmethacrylat, n-Propylmethacrylat, Isopropylmethacrylat, n-Butylmethacrylat, tert.-Butylmethacrylat
(c) Acrylsäure und/oder Methacrylsäure.

Im Rahmen einer weiteren Ausführungsform ist das erste anionische festigende Copolymer ein anionisches Copolymer, welches aus Monomeren umfassend Monomere (a) und (b1) und (b2) und (c) hergestellt wird:
(a) Styrol und/oder alpha-Methylstyrol (insbesondere Styrol),
(b1) mindestens einem Monomer ausgewählt unter Methylacrylat, Ethylacrylat, n-Propylacrylat, Isopropylacrylat, n-Butylacrylat, tert.-Butylacrylat, Methylmethacrylat, Ethylmethacrylat, n-Propylmethacrylat, Isopropylmethacrylat, n-Butylmethacrylat, tert.-Butylmethacrylat,
(b2) 2-Hydroxyethylacrylat und/oder 2-Hydroxyethylmethacrylat,
(c) Acrylsäure und/oder Methacrylsäure.

Beispiel für ein bevorzugtes erstes anionisches festigendes Polymer ist ein Polymer mit der INCI-Bezeichnung Styrene / Acrylates Copolymer.

Das neben dem ersten fixierenden Copolymer in dem erfindungsgemäßen Mittel zwingend enthaltene zweite anionische Copolymer ist vernetzt. Hierbei zeigte sich, dass der erfindungsgemäße Effekt besonders ausgeprägt war, wenn das verbrückende Polysiloxan-Molekülfragment von einem Polysiloxan abgeleitet wird, an dessen beiden Kettenenden jeweils mindestens eine radikalisch polymerisierbare Funktionalität bindet.

Das zweite vernetzte anionische Copolymer besitzt bevorzugt ein Molekulargewicht M_{w} von 35000 bis 60000 g/mol.

Die vernetzende Polysiloxan-Struktureinheit ist in dem zweiten vernetzten anionischen Copolymer in einer Menge von 0,1 bis 50,0 Gew.-%, insbesondere von 0,1 bis 40,0 Gew.-%, jeweils bezogen auf das Gewicht des Polymers, enthalten. Dies gilt für alle zuvor und später beschriebenen Ausführungsformen mutatis mutandis.

Die Menge der entsprechend im zweiten vernetzten anionischen Copolymer enthaltenen Struktureinheiten der Formel (IV) entspricht bevorzugt 30,0 bis 99,4 Gew.-%, besonders bevorzugt 10 bis 40 Gew.-%, jeweils bezogen auf das Gewicht des besagten Polymers. Dies gilt für alle zuvor und später beschriebenen Ausführungsformen mutatis mutandis.

Die Menge der entsprechend im zweiten vernetzten anionischen Copolymer enthaltenen Struktureinheiten der Formel (V) entspricht bevorzugt 0,5 bis 14,0 Gew.-%, besonders bevorzugt 0,5 bis 10,0 Gew.-%, ganz besonders bevorzugt 3,0 bis 6,0 Gew.-%, jeweils bezogen auf das Gewicht des besagten Polymers. Dies gilt für alle zuvor und später beschriebenen Ausführungsformen mutatis mutandis.

Als zweites vernetztes anionisches Copolymer werden erfindungsgemäß solche eingesetzt, welche mindestens eine vernetzende Polysiloxan-Struktureinheit der Formel (PS) und mindestens eine Struktureinheit der Formel (IV) und mindestens eine Struktureinheit der Formel (V) umfassen 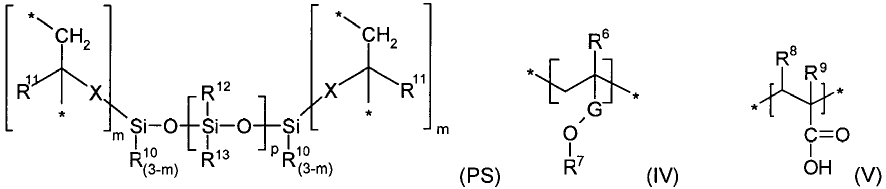 worin
- R⁶: ein Wasserstoffatom oder eine Methylgruppe bedeutet,
- R⁷: steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
- G: eine direkte Bindung oder eine Carbonylgruppe bedeutet,
- R⁸ und R⁹: stehen für ein Wasserstoffatom oder einer der beiden Reste für ein Wasserstoffatom und der andere für eine Methylgruppe,
- R¹¹: steht für ein Wasserstoffatom oder eine Methylgruppe,
- R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
- m: für eine ganze Zahl von 1 bis 3 steht
- p: für eine ganze Zahl von 10 bis 1000 steht,
- X: steht für eine direkte Bindung oder ein beliebiges Molekülfragment.

X steht gemäß Formel (PS) besonders bevorzugt für eine direkte Bindung, eine Methylengruppe, Ethan-1,2-diyl, eine Gruppe *-CO-O-(CH₂)ₓ-* worin x für 2 oder 3 steht oder eine Gruppe *-CO-NH-(CH₂)ₓ-* worin x für 2 oder 3 steht.

Si-C-gebunden bedeutet im Sinne der Erfindung, dass der besagte Kohlenwasserstoffrest über eine kovalente Silizium-Kohlenstoffbindung an das Silikonstrukturfragment gemäß Formel (PS) bindet.

m gemäß Formel (PS) steht bevorzugt für 1.

R¹⁰ gemäß Formel (PS) steht bevorzugt für eine Methylgruppe.

R¹² und R¹³ gemäß Formel (PS) stehen bevorzugt für eine Methylgruppe.

Es ist erfindungsgemäß bevorzugt, die Polysiloxan-Struktureinheit auszuwählen unter: 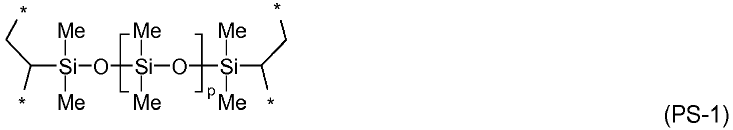 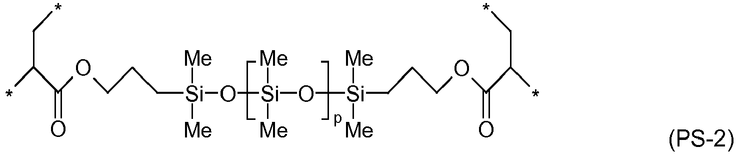 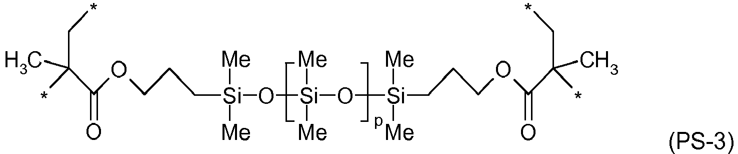 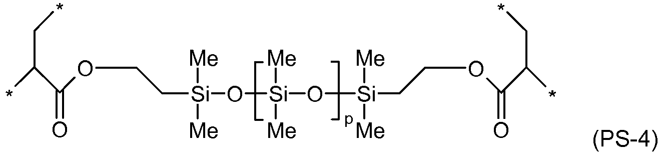 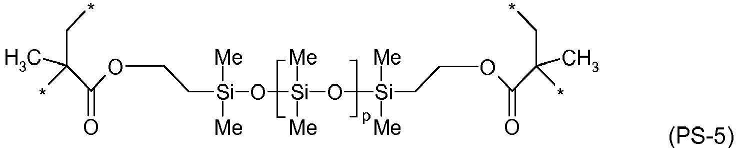 worin jeweils p für eine ganze Zahl von 10 bis 1000 steht.

Ganz besonders bevorzugte Polysiloxan-Struktureinheiten werden ausgewählt unter Formel (PS-1), (PS-2), (PS-3) (am bevorzugtesten der Formel (PS-1)).

Es stellten sich solche erfindungsgemäßen Mittel als besonders geeignet heraus, die für das zweite vernetzte anionische Copolymer mindestens eines oder mehrere der folgenden Merkmale erfüllen:
- Bevorzugt steht gemäß Formel (IV) G für eine Carbonylgruppe, R⁶ für ein Wasserstoffatom oder eine Methylgruppe und R⁷ für eine Methylgruppe, Ethylgruppe, n-Propylgruppe, Isopropylgruppe, n-Butylgruppe, sec-Butylgruppe, tert-Butylgruppe, 2-Ethylhexylgruppe.
- Bevorzugt steht gemäß Formel (IV) G für eine direkte Bindung, R⁶ für ein Wasserstoffatom und R² für eine Acetylgruppe, eine Propionylgruppe, eine verzweigte (C₈ bis C₁₈)-Alkanoylgruppe (insbesondere eine (C₈ bis C₁₈)-Isoalkanoylgruppe, bevorzugter eine (C₈ bis C₁₂)-Isoalkanoylgruppe, beispielsweise Isooctadecanoylgruppe, Isononanoylgruppe oder eine Neodecanoylgruppe).
- Bevorzugt steht R⁸ gemäß Formel (V) für eine Methylgruppe und R⁹ steht für ein Wasserstoffatom.

Bevorzugt sind daher solche erfindungsgemäßen Mittel, die in einem kosmetisch akzeptablen Träger neben dem besagten ersten festigenden anionischen Copolymer mindestens ein zweites vernetztes anionisches Copolymer enthalten, umfassend mindestens eine Struktureinheit der Formel (PS) und mindestens eine Struktureinheit der Formel (IV-1) und mindestens eine Struktureinheit der Formel (V-1) 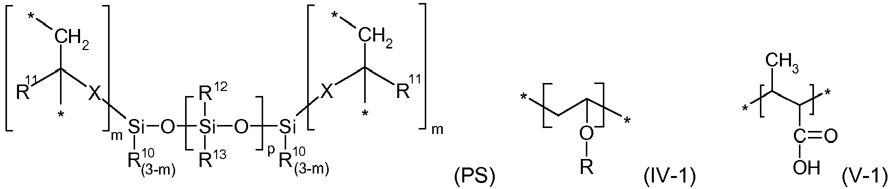 worin
- R¹¹: steht für ein Wasserstoffatom oder eine Methylgruppe,
- R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
- m: für eine ganze Zahl von 1 bis 3 steht
- p: für eine ganze Zahl von 10 bis 1000 steht,
- X: steht für eine direkte Bindung oder ein beliebiges Molekülfragment,
- R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).

Die zuvor genannten bevorzugten Bedeutungen der Gruppen R¹⁰, R¹¹, R¹², R¹³ bzw. m, p, und X gelten mutatis mutandis.

Erfindungsgemäße Mittel sind bevorzugt geeignet, in denen in einem kosmetisch akzeptablen Träger neben dem besagten ersten festigenden anionischen Copolymer mindestens ein zweites vernetztes anionisches Copolymer enthalten, umfassend mindestens eine Struktureinheit der Formel (PS-6) und mindestens eine Struktureinheit der Formel (IV-1) und mindestens eine Struktureinheit der Formel (V-1) 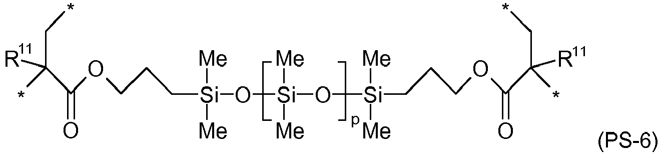 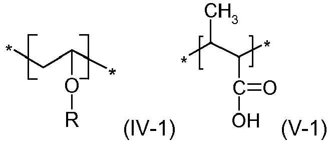 worin
- p: für eine ganze Zahl von 10 bis 1000 steht,
- R¹¹: abhängig voneinander für ein Wasserstoffatom oder eine Methylgruppe steht,
- R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).

Ferner haben sich erfindungsgemäße Mittel als besonders geeignet herausgestellt, in denen in einem kosmetisch akzeptablen Träger neben dem besagten ersten festigenden anionischen Copolymer mindestens ein zweites vernetztes anionisches Copolymer enthalten, umfassend mindestens eine Struktureinheit der Formel (PS-1) und mindestens eine Struktureinheit der Formel (IV-1) und mindestens eine Struktureinheit der Formel (V-1) 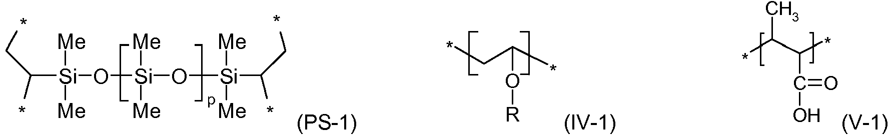 worin
- p: für eine ganze Zahl von 10 bis 1000 steht,
- R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).

Besonders bevorzugt handelt es sich bei dem zweiten anionischen vernetzen Copolymer um ein anionisches Copolymer, welches aus Monomeren umfassend Monomere (d) und (e) und (f) hergestellt wird:
(d) alpha, omega-Divinylpolydimethylsiloxan der Formel H₂C=CH-SiMe₂-O-(SiMe₂-O-)ₚ-SiMe₂-CH=CH₂ mit p = 10 bis 1000,
(e) mindestens einem Monomer ausgewählt unter Vinylacetat, Vinylpropionat, Vinylneodecanoat, Vinylisononanoat,
(f) Crotonsäure und/oder Acrylsäure und/oder Methacrylsäure (insbesondere Crotonsäure).

Im Rahmen einer weiteren Ausführungsform ist das zweite anionische vernetze Copolymer ein anionisches Copolymer, welches aus Monomeren umfassend Monomere (d) und (e1) und (e2) und (f) hergestellt wird:
(d) alpha, omega-Divinylpolydimethylsiloxan der Formel H₂C=CH-SiMe₂-O-(SiMe₂-O-)ₚ-SiMe₂-CH=CH₂ mit p = 10 bis 1000,
(e1) mindestens einem Monomer ausgewählt unter Vinylacetat, Vinylpropionat
(e2) mindestens einem Monomer ausgewählt unter Vinylneodecanoat, Vinylisononanoat,
(f) Crotonsäure und/oder Acrylsäure und/oder Methacrylsäure (insbesondere Crotonsäure).

Die erfindungsgemäßen zweiten anionischen vernetzen Copolymere sind beispielsweise unter dem Handelsnamen Wacker Belsil^{®} P101 von der Firma Wacker Chemie AG erhältlich.

Ganz besonders geeignete erfindungsgemäße Mittel sind die nachfolgend genannten Mittel (B) bis (L) beispielhaft genannt:
(B): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend mindestens eine Struktureinheit der Formel (I) und mindestens eine Struktureinheit der Formel (II) und mindestens eine Struktureinheit der Formel (III) 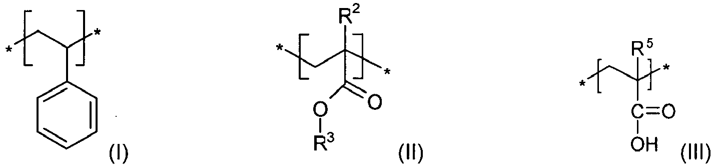 worin
      R² steht für ein Wasserstoffatom oder eine Methylgruppe
      R³ steht für eine (C₁ bis C₂₀)-Alkylgruppe, oder eine (C₂ bis C₆)-Hydroxyalkylgruppe, und
      R⁵ steht für ein Wasserstoffatom oder eine Methylgruppe.
      und
   - mindestens ein zweites vernetztes anionisches Copolymer umfassend mindestens eine Struktureinheit der Formel (PS) und mindestens eine Struktureinheit der Formel (IV-1) und mindestens eine Struktureinheit der Formel (V-1) 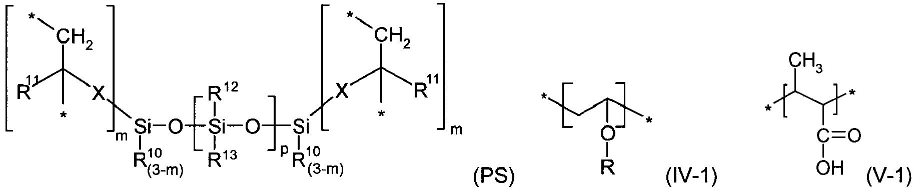 worin
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(C): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend mindestens eine Struktureinheit der Formel (I) und mindestens eine Struktureinheit der Formel (II) und mindestens eine Struktureinheit der Formel (III) 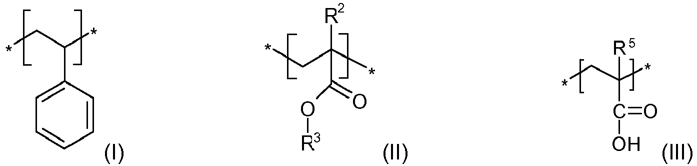 worin
      R² steht für ein Wasserstoffatom oder eine Methylgruppe
      R³ steht für eine (C₁ bis C₂₀)-Alkylgruppe, oder eine (C₂ bis C₆)-Hydroxyalkylgruppe, und
      R⁵ steht für ein Wasserstoffatom oder eine Methylgruppe.
      und
   - mindestens ein zweites vernetztes anionisches Copolymer umfassend umfassend mindestens eine Struktureinheit der Formel (PS-1) und mindestens eine Struktureinheit der Formel (IV-1) und mindestens eine Struktureinheit der Formel (V-1) 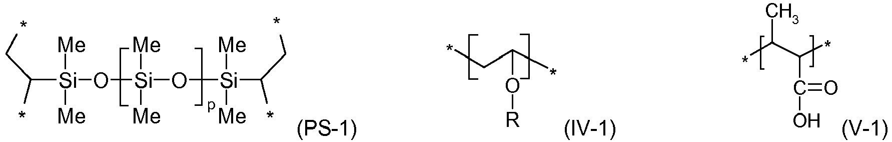 worin
      - p: für eine ganze Zahl von 10 bis 1000 steht,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(D): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend mindestens eine Struktureinheit der Formel (I) und mindestens eine Struktureinheit der Formel (IIa) und mindestens eine Struktureinheit der Formel (IIb) und mindestens eine Struktureinheit der Formel (III) 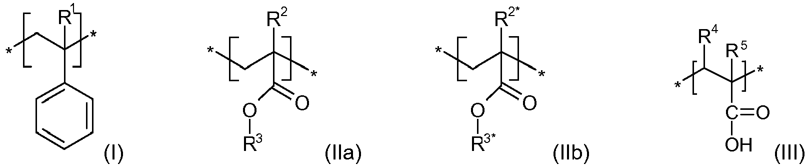 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH₂O)ₙ(C₁ bis C₂₀)-Alkyl mit n = 1 bis 30,
      R² steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend mindestens eine vernetzende Polysiloxan-Struktureinheit der Formel (PS) und mindestens eine Struktureinheit der Formel (IV) und mindestens eine Struktureinheit der Formel (V) 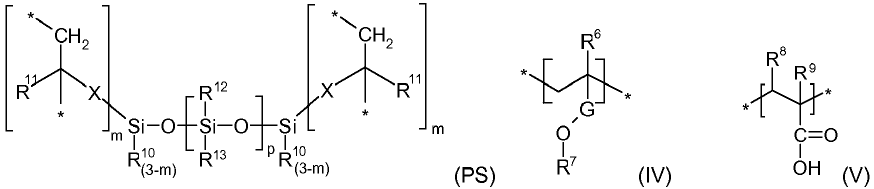 worin
      - R⁶: ein Wasserstoffatom oder eine Methylgruppe bedeutet,
      - R⁷: steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
      - G: eine direkte Bindung oder eine Carbonylgruppe bedeutet,
      - R⁸ und R⁹: stehen für ein Wasserstoffatom oder einer der beiden Reste für ein Wasserstoffatom und der andere für eine Methylgruppe,
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment.
(E): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend mindestens eine Struktureinheit der Formel (I) und mindestens eine Struktureinheit der Formel (IIa) und mindestens eine Struktureinheit der Formel (IIb) und mindestens eine Struktureinheit der Formel (III) 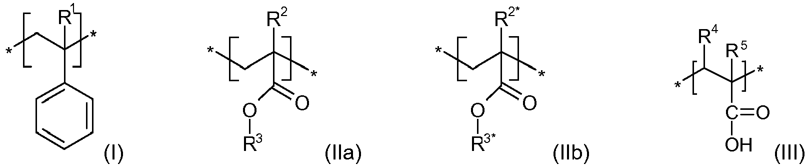 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH₂O)ₙ-(C₁ bis C₂₀)-Alkyl mit n = 1 bis 30,
      R² steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend mindestens eine Struktureinheit der Formel (PS) und mindestens eine Struktureinheit der Formel (IV-1) und mindestens eine Struktureinheit der Formel (V-1) 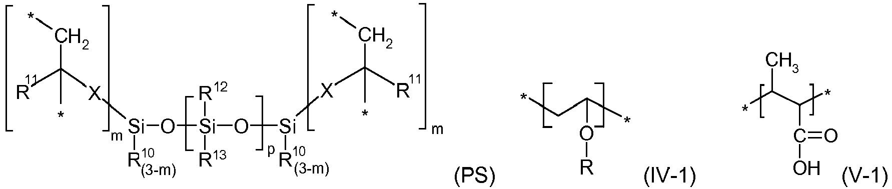 worin
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(F): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend mindestens eine Struktureinheit der Formel (I) und mindestens eine Struktureinheit der Formel (IIa) und mindestens eine Struktureinheit der Formel (IIb) und mindestens eine Struktureinheit der Formel (III) 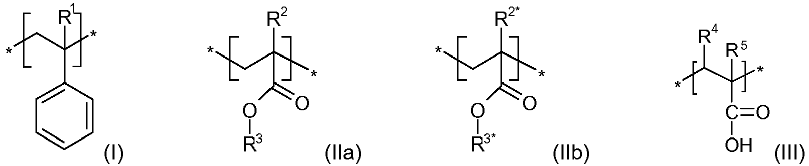 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH_{2O})ₙ-(C₁ bis C₂₀)-Alkyl mit n = 1 bis 30,
      R² steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend mindestens eine Struktureinheit der Formel (PS-1) und mindestens eine Struktureinheit der Formel (IV-1) und mindestens eine Struktureinheit der Formel (V-1) 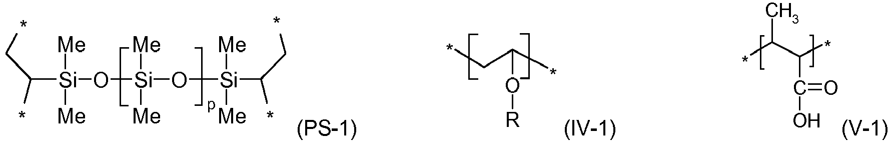 worin
      p für eine ganze Zahl von 10 bis 1000 steht,
      R steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(G): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% mindestens einer Struktureinheit der Formel (II) und 5 bis 50 Gew.-% mindestens einer Struktureinheit der Formel (III) 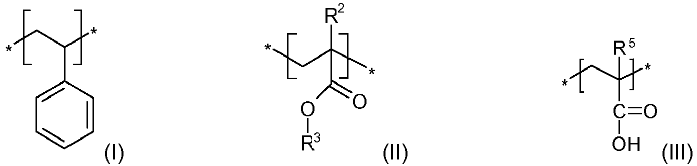 worin
      R² steht für ein Wasserstoffatom oder eine Methylgruppe
      R³ steht für eine (C₁ bis C₂₀)-Alkylgruppe, oder eine (C₂ bis C₆)-Hydroxyalkylgruppe, und
      R⁵ steht für ein Wasserstoffatom oder eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS) und 30 bis 99,4 Gew.-% mindestens einer Struktureinheit der Formel (IV) und 0,5 bis 14,0 Gew.-% mindestens einer Struktureinheit der Formel (V) 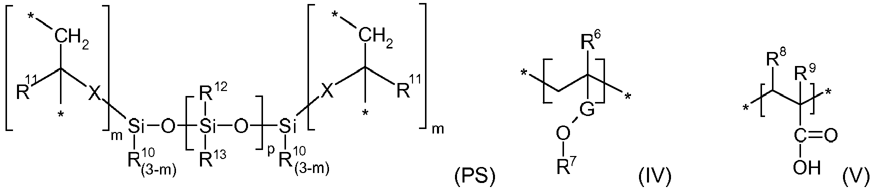 worin
      - R⁶: ein Wasserstoffatom oder eine Methylgruppe bedeutet,
      - R⁷: steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
      - G: eine direkte Bindung oder eine Carbonylgruppe bedeutet,
      - R⁸ und R⁹: stehen für ein Wasserstoffatom oder einer der beiden Reste für ein Wasserstoffatom und der andere für eine Methylgruppe,
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment.
(H): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% mindestens einer Struktureinheit der Formel (II) und 5 bis 50 Gew.-% mindestens einer Struktureinheit der Formel (III) 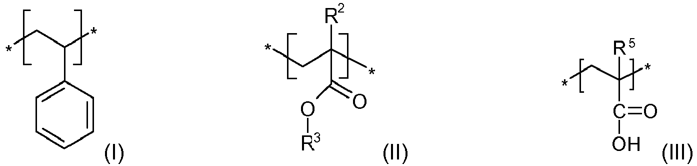 worin
      R² steht für ein Wasserstoffatom oder eine Methylgruppe
      R³ steht für eine (C₁ bis C₂₀)-Alkylgruppe, oder eine (C₂ bis C₆)-Hydroxyalkylgruppe, und
      R⁵ steht für ein Wasserstoffatom oder eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS) und 30 bis 99,4 Gew.-% mindestens einer Struktureinheit der Formel (IV-1) und 0,5 bis 14,0 Gew.-% mindestens einer Struktureinheit der Formel (V-1) 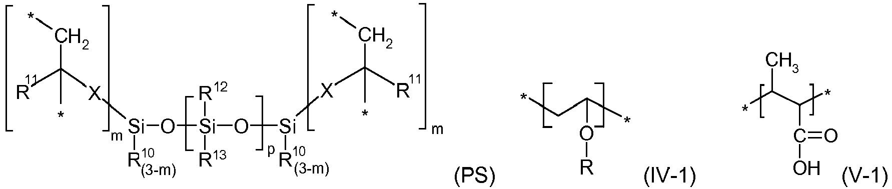 worin
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(I): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% mindestens einer Struktureinheit der Formel (II) und 5 bis 50 Gew.-% mindestens einer Struktureinheit der Formel (III) 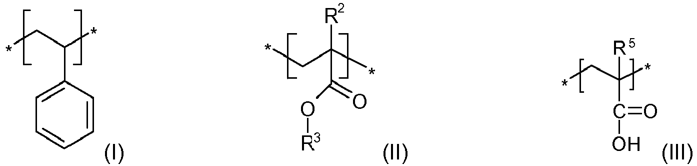 worin
      R² steht für ein Wasserstoffatom oder eine Methylgruppe
      R³ steht für eine (C₁ bis C₂₀)-Alkylgruppe, oder eine (C₂ bis C₆)-Hydroxyalkylgruppe, und
      R⁵ steht für ein Wasserstoffatom oder eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS-1) und 30 bis 99,4 Gew.-% mindestens einer Struktureinheit der Formel (IV-1) und 0,5 bis 14,0 Gew.-% mindestens einer Struktureinheit der Formel (V-1) 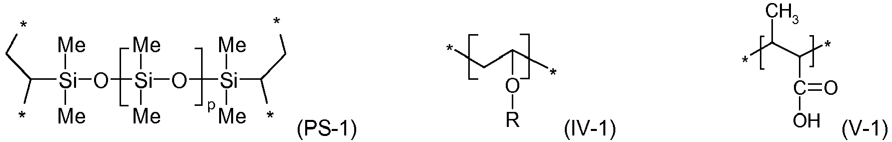 worin
      - p: für eine ganze Zahl von 10 bis 1000 steht,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(J): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% in Summe mindestens einer Struktureinheit der Formel (IIa) und (IIb) und 5 bis 50 Gew.-% mindestens einer Struktureinheit der Formel (III) 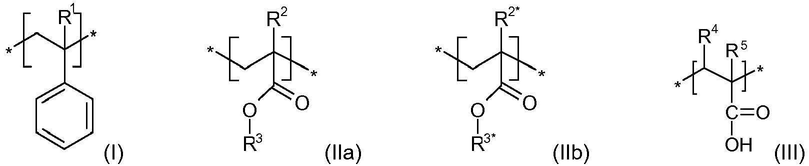 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH₂O)ₙ-(C₁ bis C₂₀)-Alkyl mit n = 1 bis 30,
      R² steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS) und 30 bis 99,4 Gew.-% mindestens einer Struktureinheit der Formel (IV-1) und 0,5 bis 14,0 Gew.-% mindestens einer Struktureinheit der Formel (V-1) 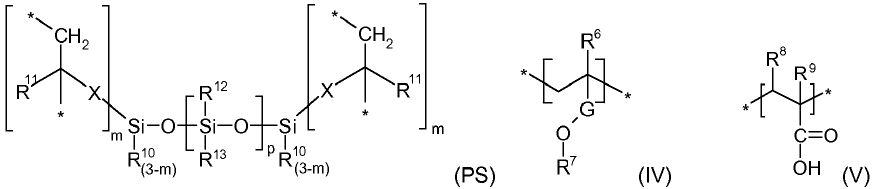 worin
      - R⁶: ein Wasserstoffatom oder eine Methylgruppe bedeutet,
      - R⁷: steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
      - G: eine direkte Bindung oder eine Carbonylgruppe bedeutet,
      - R⁸ und R⁹: stehen für ein Wasserstoffatom oder einer der beiden Reste für ein Wasserstoffatom und der andere für eine Methylgruppe,
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment.
(K): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% in Summe mindestens einer Struktureinheit der Formel (IIa) und (IIb) und 5 bis 50 Gew.-% mindestens einer Struktureinheit der Formel (III) 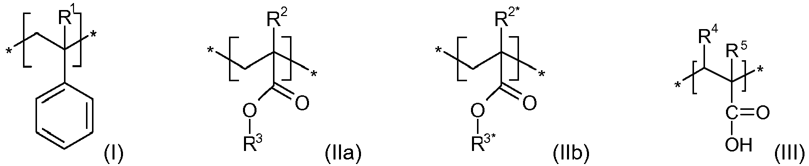 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH₂O)ₙ-(C₁ bis C₂₀)-Alkyl mit n = 1 bis 30,
      R² steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS) und 30 bis 99,4 Gew.-% mindestens einer Struktureinheit der Formel (IV-1) und 0,5 bis 14,0 Gew.-% mindestens einer Struktureinheit der Formel (V-1) 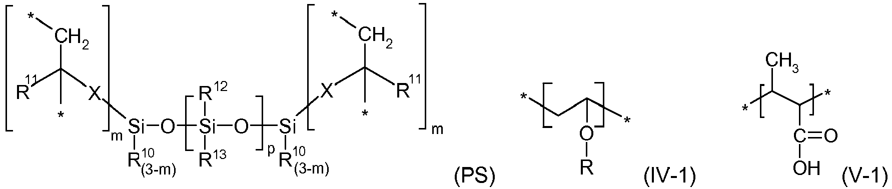 worin
      - R¹¹: steht für ein Wasserstoffatom oder eine Methylgruppe,
      - R¹⁰, R¹², R¹³: stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
      - m: für eine ganze Zahl von 1 bis 3 steht
      - p: für eine ganze Zahl von 10 bis 1000 steht,
      - X: steht für eine direkte Bindung oder ein beliebiges Molekülfragment,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).
(L): Mittel zur Behandlung keratinhaltiger Fasern, insbesondere menschlichem Haar, enthaltend in einem kosmetisch akzeptablen Träger
   - mindestens ein erstes festigendes anionisches Copolymer, umfassend jeweils bezogen auf das Gewicht des Polymers 30 bis 60 Gew.-% mindestens einer Struktureinheit der Formel (I) und 1 bis 50 Gew.-% in Summe mindestens einer Struktureinheit der Formel (IIa) und (IIb) und 5 bis 50 Gew.-% mindestens einer Struktureinheit der Formel (III) 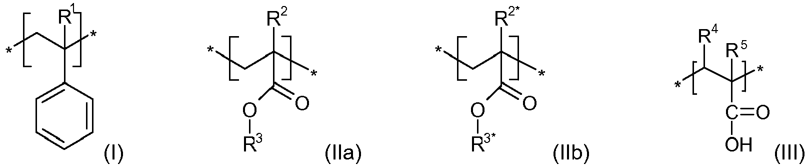 worin
      R¹ steht für ein Wasserstoffatom oder eine Methylgruppe,
      R² steht für ein Wasserstoffatom oder eine Methylgruppe
      R³ steht für eine (C₁ bis C₂₀)-Alkylgruppe oder eine Gruppe *-(CH₂CH₂O)ₙ-(C₁ bis C₂₀)-Alkyl mit n = 1 bis 30,
      R^{2*} steht für ein Wasserstoffatom oder eine Methylgruppe
      R^{3*} steht für eine (C₂ bis C₆)-Hydroxyalkylgruppe,
      R⁴ und R⁵ stehen für ein Wasserstoffatom oder einer der beiden Reste für ein Wasserstoffatom und der andere für eine Methylgruppe
      und
   - mindestens ein zweites vernetztes anionisches Copolymer umfassend jeweils bezogen auf das Gewicht des Polymers 0,1 bis 50 Gew.-% mindestens einer vernetzenden Polysiloxan-Struktureinheit der Formel (PS-1) und 30 bis 99,4 Gew.-% mindestens einer Struktureinheit der Formel (IV-1) und 0,5 bis 14,0 Gew.-% mindestens einer Struktureinheit der Formel (V-1) 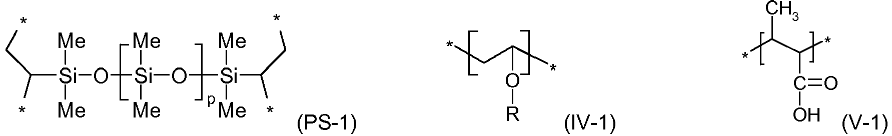 worin
      - p: für eine ganze Zahl von 10 bis 1000 steht,
      - R: steht für eine lineare oder verzweigte (C₂ bis C₁₈)-Acylgruppe (insbesondere für (C₈ bis C₁₂)-Isoalkanoyl, Isononanoyl, Neodecanoyl, Acetyl, Propionyl).

Alle zuvorgenannten bevorzugten Parameter gelten mutatis mutandis auch für die beispielhaft genannten besonders geeigneten Ausführungsformen (B) bis (L).

Die erfindungsgemäßen Mittel enthalten die Inhalts- bzw- Wirkstoffe in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Im Rahmen einer bevorzugten Ausführungsform des erfindungsgemäßen Mittel enthält das Mittel daher zusätzlich mindestens einen Alkohol, der 2 bis 6 Kohlenstoffatome und 1 bis 3 Hydroxylgruppen aufweist. Dieser zusätzliche Alkohol wird wiederum bevorzugt ausgewählt aus mindestens einer Verbindung der Gruppe, die gebildet wird aus Ethanol, Ethylenglykol, Isopropanol, 1,2-Propylenglykol, 1,3-Propylenglykol, Glyzerin, n-Butanol, 1,3-Butylenglykol. Ein ganz besonders bevorzugter Alkohol ist Ethanol.

Der zusätzliche Alkohol mit 2 bis 6 Kohlenstoffatomen und 1 bis 3 Hydroxylgruppen ist bevorzugt in dem erfindungsgemäßen Mittel (insbesondere in Gegenwart mindestens eines Treibmittels) in einer Menge von 40 Gew.-% bis 65 Gew.-%, insbesondere von 40 Gew.-% bis 50 Gew.-%, jeweils bezogen auf das Gewicht des kosmetischen Mittels, enthalten.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Polyethylenglykol und Propylenglykol in einer Menge bis 30 Gew.-% bezogen auf das gesamte Mittel.

Insbesondere der Zusatz von Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Effekte liessen sich durch Zusatz mindestens eines (C₂ bis C₆)-Trialkylcitrats zum erfindungsgemäßen Mittel steigern. Daher ist es erfindungsgemäß bevorzugt, wenn die Mittel zusätzlich mindestens eine Verbindung der Formel (E) enthalten, 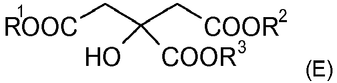 worin
R¹, R² und R³ unabhängig voneinander für eine (C₂ bis C₆)-Alkylgruppe stehen.

Beispiele einer (C₂ bis C₆)-Alkylgruppe gemäß Formel (E) sind Methyl, Ethyl, Isopropyl, n-Propyl, n-Butyl, sec-Butyl, Isobutyl, tert-Butyl, n-Pentyl, Neopentyl, Isopentyl, n-Hexyl.
Als besonders bevorzugte Verbindung der Formel (E) stellte sich Triethylcitrat heraus.
Das erfindungsgemäße Mittel enthält die Verbindungen der Formel (E) bevorzugt in einer Menge von 0,01 bis 1 Gew.-%, insbesondere von 0,05 bis 0,3 Gew.-%, jeweils bezogen auf das Gewicht des gesamten Mittels.

Bevorzugt enthalten die erfindungsgemäßen Mittel vorzugsweise zusätzlich mindestens ein Tensid, wobei sich insbesondere nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben. Der Einsatz mindestens eines nichtionischen Tensids und/oder mindestens eines kationischen Tensids ist im Rahmen dieser Ausführungsform der Erfindung bevorzugt.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (T-I)

   R¹CO-(OCH₂CHR²)_{w}OR³ (T-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (T-II),

   R⁴O-[G]ₚ (T-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die bevorzugten Alkyl- und/oder Alkenyloligoglykoside sind Alkyl- und/oder Alkenyloligoglucoside. Die Indexzahl p in der allgemeinen Formel (T-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Bevorzugt sind Alkyloligoglucoside auf Basis von gehärtetem C_{12/14}-Kokosalkohol mit einem DP von 1 bis 3.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin^{®} CS 15 (COGNIS), Cremophor A25 (BASF SE) bzw. Eumulgin^{®} CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH2-CH2O)x-CH2-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (T-V), 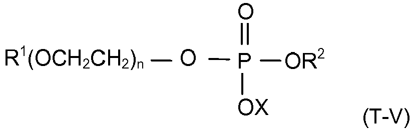 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (T-VI)

   R⁷CO(AlkO)ₙSO₃M (T-VI)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Die erfindungsgemäßen Mittel können optional zusätzlich mindestens ein filmbildendes Polymer und/oder festigendes Polymer enthalten. Diese zusätzlichen Polymere sind von dem ersten filmbildenden anionischen Copolymer und dem zweiten vernetzten anionischen Copolymer verschieden. Die optional zugesetzten filmbildenden und/oder festigenden Polymere sind bevorzugterweise kationisch und/oder nichtionisch.

Die kationischen filmbildenden und/oder kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Erfindung vorteilhaft, die in einer Seitenkette mehr als eine permanente kationische Ladung tragen.

Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Weiterhin eignen sich solche kationischen filmbildenden und/oder kationischen festigenden Polymere, die mindestens eine Struktureinheit der Formel (M-I) und mindestens eine Struktureinheit der Formel (M-VI) und gegebenenfalls mindestens eine Struktureinheit der Formel (M-V) umfassen 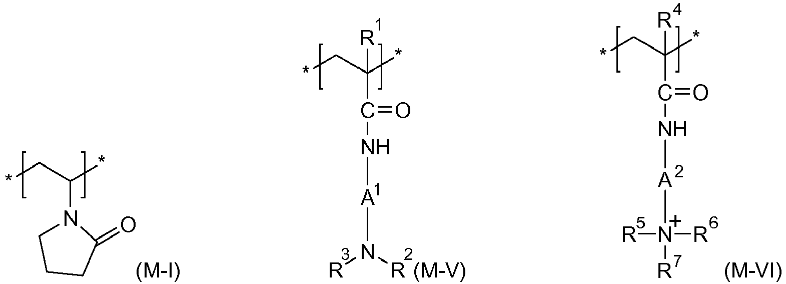 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (M-VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Geeignete Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit Vinylpyrrolidon und Dimethylaminopropylmethacrylamid mit der INCI-Bezeichnung Polyquaternium-55 unter den Handelsnamen, Styleze^{®} W-10, Styleze^{®} W-20 (Firma ISP),
- Copolymere aus N-Vinylpyrrolidon, N-Vinylcaprolactam, N-(3-Dimethylaminopropyl)-methacrylamid und 3-(Methacryloylamino)propyl-lauryl-dimethylammoniumchlorid (INCI-Bezeichnung: Polyquaternium-69) unter dem Handelsnamen AquaStyle^{®} 300 (28-32 Gew.-% Aktivsubstanz in Ethanol-Wasser-Gemisch) (Firma ISP)
im Handel erhältlich.

Als im Sinne der Erfindung besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Polymere, die mindestens ein Strukturelement der Formel (M1) enthalten 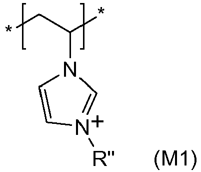 worin
R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung der Komponente dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es ist wiederum erfindungsgemäß bevorzugt, wenn in der erfindungsgemäßen pulverförmigen Zusammensetzung als kationisches filmbildendes und/oder kationisches festigendes Polymer dieser Ausführungsform, mindestens ein Copolymer (b1) enthalten ist, das neben mindestens einem Strukturelement der Formel (M1) zusätzlich ein Strukturelement der Formel (M-I) umfasst 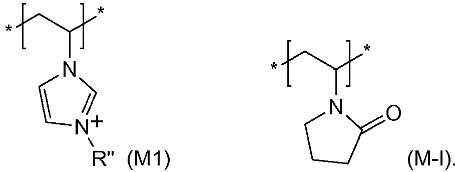 worin
R" für eine (C₁ bis C₄)-Alkylgruppe, insbesondere eine Methylgruppe, steht.

Zur Kompensation der positiven Polymerladung der Copolymere (b1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (b1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (M-I).

Hierbei ist besonders bevorzugt, wenn die Copolymere (b1) neben Polymereinheiten, die aus dem Einbau der genannten Struktureinheiten gemäß Formel (M1) und (M-I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b1) ausschließlich aus Struktureinheiten der Formel (M1) mit R" = Methyl und (M-I) aufgebaut und lassen sich durch die allgemeine Formel (Poly1) 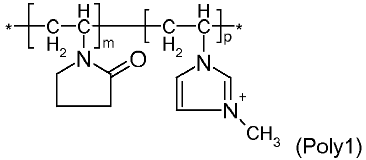 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (M-I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552
Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Zusätzlich zu dem bzw. den Copolymer(en) (b1) oder an dessen bzw. deren Stelle können erfindungsgemäße pulverförmige Zusammensetzungen auch Copolymere (b2) enthalten, die ausgehend vom Copolymer (b1) als zusätzliche Struktureinheiten Struktureinheiten der Formel (M-II) aufweisen

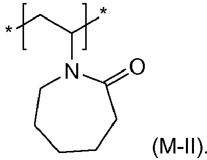

Weitere besonders bevorzugte erfindungsgemäße pulverförmige Zusammensetzungen sind somit dadurch gekennzeichnet, daß sie als kationisches filmbildendes und/oder kationisches festigendes Polymer mindestens ein Copolymer (b2) enthalten, das mindestens eine Struktureinheit gemäß Formel (M1-a) und mindestens eine Struktureinheit gemäß Formel (M-I) und mindestens eine Struktureinheit gemäß Formel (M-II) enthält

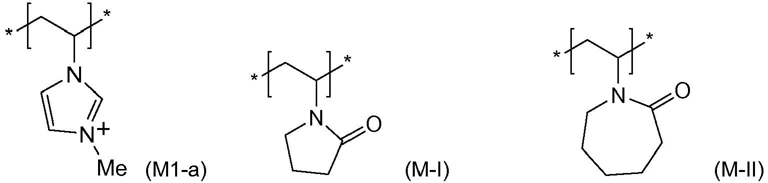

Auch hierbei ist besonders bevorzugt, wenn die Copolymere (b2) neben Polymereinheiten, die aus dem Einbau der genannten Struktureinheiten gemäß Formel (M1-a), (M-I) und (M-II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b2) ausschließlich aus Struktureinheiten der Formeln (M1-a), (M-I) und (M-II) aufgebaut und lassen sich durch die allgemeine Formel (Poly2) 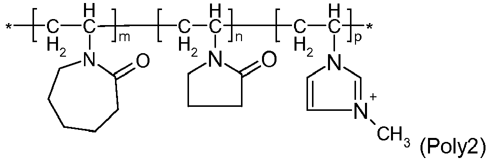 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (b2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (M-II).

Zusätzlich zu dem bzw. den Copolymer(en) (b1) und/oder (b2) oder an dessen bzw. deren Stelle können die erfindungsgemäßen pulverförmigen Zusammensetzungen als filmbildendes kationische und/oder festigendes kationisches Polymer auch Copolymere (b3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße pulverförmige Zusammensetzungen sind dadurch gekennzeichnet, daß sie als kationisches filmbildendes und/oder kationisches festigendes Polymer mindestens ein Copolymer (b3) enthalten, das mindestens eine Struktureinheit gemäß Formel (M1-a) und mindestens eine Struktureinheit gemäß Formel (M-I) und mindestens eine Struktureinheit gemäß Formel (M-VII) und mindestens eine Struktureinheit gemäß Formel (M-VIII) enthält

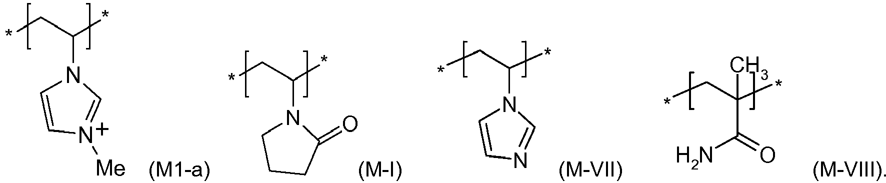

Auch hierbei ist besonders bevorzugt, wenn die Copolymere (b3) neben Polymereinheiten, die aus dem Einbau der genannten Struktureinheiten gemäß Formel (M1-a), (M-I), (M-VII) und (M-VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b3) ausschließlich aus Struktureinheiten der Formel (M1-a), (M-I), (M-VII) und (M-VIII) aufgebaut und lassen sich durch die allgemeine Formel (Poly3) 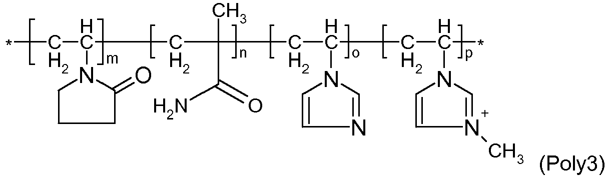 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (M-I), (M-VII) und (M-VIII) im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (b3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (M-I) und 1 bis 20 Mol.-%, vorzugsweise 5 bis 15 Mol.-% und insbesondere 10 Mol.-% Struktureinheiten gemäß Formel (M-VII) und 20 bis 40 Mol.-%, vorzugsweise 25 bis 35 Mol.-% und insbesondere 29 Mol.-% Struktureinheiten gemäß Formel (M-VIII).

Unter den zusätzlichen filmbildenden kationischen und/oder festigenden Polymer ausgewählt aus den kationischen Polymeren mit mindestens einem Strukturelement der obigen Formel (M1), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren.

Im Rahmen einer bevorzugten Ausführungsform enthalten die erfindungsgemäßen pulverförmigen Zusammensetzungen als filmbildendes und/oder festigendes Polymer mindestens ein filmbildendes nichtionisches und/oder festigendes nichtionisches Polymer.

Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 5,0 Gew.-%, jeweils bezogen auf das Gewicht der erfindungsgemäßen pulverförmigen Zusammensetzung, enthalten.

Die filmbildenden nichtionischen und/oder festigenden nichtionischen Polymere werden wiederum bevorzugt ausgewählt aus mindestens einem Polymer der Gruppe, die gebildet wird, aus
- Homopolymeren und nichtionischen Copolymeren des N-Vinylpyrrolidons,
- nichtionischen Copolymeren des Isobutens,
- nichtionischen Copolymeren des Maleinsäureanhydrids.

Dabei ist eine Kombination von filmbildenden nichtionischen und/oder festigenden nichtionischen Polymeren bevorzugt, umfassend mindestens ein nichtionisches Copolymer des Maleinsäureanhydrids und mindestens ein Polymer aus der Gruppe, die gebildet wird aus Homopolymeren und nichtionischen Copolymeren des N-Vinylpyrrolidons.

Geeignete Polyvinylpyrrolidone sind beispielsweise Handelsprodukte wie Luviskol^{®} K 90 oder Luviskol^{®} K 85 der Firma BASF SE.
Geeignete Polyvinylalkohole werden beispielsweise unter den Handelsbezeichnungen Elvanol^{®} von Du Pont oder Vinol^{®} 523/540 von der Firma Air Products vertrieben.
Geeignetes Polyvinylacetat wird beispielsweise unter dem Handelsnamen Vinac^{®} als Emulsion von der Firma Air Products vertrieben.

Mittel, die als filmbildendes nichtionisches und/oder festigendes nichtionisches Polymer mindestens ein Polymer ausgewählt aus der Gruppe, die gebildet wird aus
- Copolymer aus Maleinsäureanhydrid und Methylvinylether,
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid,
oder Gemische aus diesen Polymeren enthalten, sind erfindungsgemäß ganz besonders bevorzugt.

Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Weitere bevorzugte erfindungsgemäße pulverförmige Zusammensetzungen sind dadurch gekennzeichnet, daß sie als nichtionisches filmbildendes und/oder nichtionisches festigendes Polymer mindestens ein Copolymer (n1) enthalten, das mindestens eine Struktureinheit gemäß Formel (M-I) und mindestens eine Struktureinheit gemäß Formel (M-VII) enthält und mindestens eine Struktureinheit gemäß Formel (M-VIII) enthält

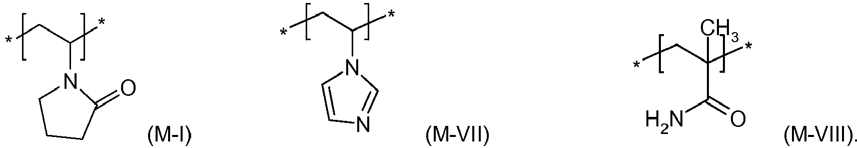

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (n1) ausschließlich aus Struktureinheiten der Formel (M1-a), (I), (VII) und (VIII) aufgebaut und lassen sich durch die allgemeine Formel (Poly4) 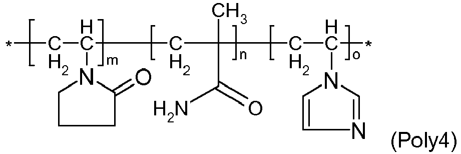 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Weiterhin eignen sich erfindungsgemäß solche pulverförmigen Zusammensetzungen, die mindestens ein nichtionisches filmbildendes und/oder nichtionisches festigendes Polymer enthalten, umfassend mindestens eine Struktureinheit der Formel (M-I) und mindestens eine Struktureinheit der Formel (M-III) 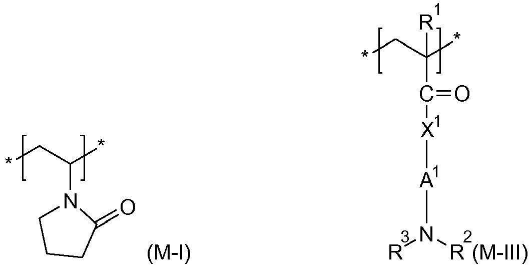 worin
R¹ für ein Wasserstoffatom oder eine Methylgruppe steht,
X¹ für ein Sauerstoffatom oder eine Gruppe NH steht,
A¹ für eine Gruppe Ethan-1,2-diyl, Propan-1,3-diyl oder Butan-1,4-diyl steht
R² und R³ stehen unabhängig voneinander für eine (C₁ bis C₄)-Alkylgruppe.

Dabei ist es insbesondere bevorzugt, wenn das obige nichtionische filmbildende und/oder nichtionische festigende Polymer ausgewählt wird aus mindestens einem Polymer, welches mindestens eines oder mehrere der folgenden Merkmale erfüllt:
- R¹ bedeutet eine Methylgruppe,
- X¹ steht für eine Gruppe NH,
- A¹ steht für Ethan-1,2-diyl oder Propan-1,3-diyl,
- R² und R³, stehen unabhängig voneinander für Methyl oder Ethyl, (besonders bevorzugt für Methyl).

Besonders bevorzugt handelt es sich bei dem nichtionischen filmbildenden und/oder nichtionischen festigenden Polymer dieser Ausführungsform um mindestens ein Polymer, das mindestens eine Struktureinheit der Formel (M-I) und mindestens eine Struktureinheit der Formel (M-III-8) umfasst,

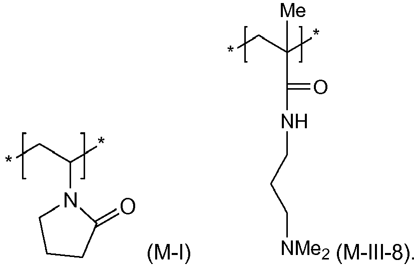

Ein ganz besonders bevorzugtes nichtionisches filmbildendes und/oder nichtionisches festigendes Polymer dieser Ausführungsform ist ein Copolymer aus N-Vinylpyrrolidon und N,N-Dimethylaminiopropylmethacrylamid, welches beispielsweise mit der INCI-Bezeichnung VP/DMAPA Acrylates Copolymer z.B. unter der Handelsbezeichnung Styleze^{®} CC 10 von der Firma ISP verkauft wird.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Selbstverständlich können erfindungsgemäß auch ß-Aminosäuren und deren Derivate eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.
Erfindungsgemäß können Proteinhydrolysate sowohl pflanzlichen als auch tierischen oder marinen oder synthetischen Ursprungs eingesetzt werden.
Wenngleich der Einsatz der Proteinhydrolysate als solche bevorzugt ist, können an deren Stelle gegebenenfalls auch anderweitig erhaltene Aminosäuregemische eingesetzt werden.

Die Proteinhydrolysate können in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten sein.

Als Pflegestoff kann das erfindungsgemäße Mittel weiterhin mindestens ein Vitamin, ein Provitamin, eine Vitaminvorstufe und/oder eines derer Derivate enthalten.
Dabei sind erfindungsgemäß solche Vitamine, Provitamine und Vitaminvorstufen bevorzugt, die üblicherweise den Gruppen A, B, C, E, F und H zugeordnet werden.
Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Weiterhin kann es bevorzugt sein, in den erfindungsgemäßen Mitteln Mischungen aus mehreren, insbesondere aus zwei, verschiedenen Pflanzenextrakten einzusetzen. Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Bevorzugt sind solche UV-Filter, deren molarer Extinktionskoeffizient am Absorptionsmaximum oberhalb von 15 000, insbesondere oberhalb von 20 000, liegt.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.
Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.
Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ganz besonders bevorzugte kationische direktziehende Farbstoffe der Gruppe (c) sind die unter den Bezeichnungen Basic Yellow 87, Basic Orange 31 und Basic Red 51 bekannten Farbstoffe. Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Mittel können neben den genannten Komponenten weiterhin alle für solche Zubereitungen bekannten Wirk-, Zusatz- und Hilfsstoffe enthalten.
Weitere Wirk-, Hilfs- und Zusatzstoffe, sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate,
- Strukturanten wie Maleinsäure und Milchsäure,
- Parfümöle, Dimethylisosorbid und Cyclodextrine,
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Konsistenzgeber wie Zuckerester, Polyolester oder Polyolalkylether,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für Stylingmittel üblichen Formen erfolgen, beispielsweise in Form von Lösungen, die als Lotion oder Pump- oder Aerosolspray auf das Haar aufgebracht werden können, oder anderen Zubereitungen, die für die Anwendung auf dem Haar geeignet sind.
Vorzugsweise werden die erfindungsgemäßen Mittel als Pumpspray, Aerosolspray, Pumpschaum oder Aerosolschaum ausgestaltet. Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt. Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pump- oder Quetschsystem verteilt wird.
Insbesondere bevorzugt liegen die erfindungsgemäßen Mittel als Aerosolhaarschaum oder Aerosolhaarspray vor. Das erfindungsgemäße Mittel enthält daher bevorzugt zusätzlich mindestens ein Treibmittel.

Erfindungsgemäße Mittel, die in Form eines Aerosolprodukts vorliegen, lassen sich in üblicher Art und Weise herstellen. In der Regel werden alle Bestandteile des erfindungsgemäßen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

In der Ausführungsform als Aerosolspray sind erfindungsgemäß geeignete Treibmittel beispielsweise ausgewählt aus N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Das Treibmittel ist in den erfindungsgemäßen Mitteln der Ausführungsform als Aerosolspray bevorzugt in einer Menge von 30 bis 60 Gew.-% - bezogen auf das Gewicht des gesamten Mittels - enthalten.
Ganz besonders bevorzugt werden Mischungen von Propan und Butan als alleiniges Treibmittel verwendet im Gewichtsverhältnis Propan zu Butan von 20 zu 80 bis 15 zu 85. Diese Mischungen werden wiederum bevorzugt in den erfindungsgemäßen Mitteln in einer Menge von 30 bis 55 Gew.-% - bezogen auf das Gewicht des gesamten Mittels - eingesetzt. Unter Butan wird erfindungsgemäß n-Butan, iso-Butan und Gemische aus n-Butan und iso-Butan verstanden.

Über das Mengenverhältnis von Treibmittel zu den übrigen Bestandteilen der Zubereitungen lassen sich bei gegebener Sprühvorrichtung die Größen der Aerosoltröpfchen und die jeweilige Größenverteilung einstellen.

Die Sprührate der erfindungsgemäßen Sprays beträgt bevorzugt 6,5 bis 10,0 g/10 s.

Besonders bevorzugte erfindungsgemäße Mittel (Aerosolsprays) sind in einem Aerosolbehälter mit einem Stem-Ventil mit einer Stembohrung mit 0,27 bis 0,35 mm Durchmesser konfektioniert. Solche Ventile werden beispielsweise als Ventile des Typs KE oder des Typs KEN von der Fa. Coster vertrieben.

In der Ausführungsform als Aerosolschaum sind erfindungsgemäß geeignete Treibmittel beispielsweise ausgewählt aus N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Die Erfindung umfasst aber ausdrücklich auch die Mitverwendung von Treibmitteln vom Typ der Fluorchlorkohlenwasserstoffe, insbesondere aber der Fluorkohlenwasserstoffe. Gemäß der Ausführungsform eines Aerosolschaums werden die genannten Alkane, Mischungen der genannten Alkane oder Mischungen der genannten Alkane mit Dimethylether als einziges Treibmittel bevorzugt eingesetzt. Besonders bevorzugte Treibmittel sind Dimethylether, Propan, n-Butan, iso-Butan und Mischungen daraus.

Über das Mengenverhältnis von Treibmittel zu den übrigen Bestandteilen der Zubereitungen lassen sich bei gegebener Sprühvorrichtung die Größen der Schaumblasen und die jeweilige Größenverteilung einstellen.

Bei Verwendung herkömmlicher Aerosolbehälter enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus N₂O, Dimethylether, CO₂, Luft, Alkanen mit 3 oder 4 Kohlenstoffatomen (wie Propan, n-Butan, iso-Butan) sowie Mischungen daraus.

Der Einsatz der zuvor genannten zusätzlichen bevorzugten Inhaltsstoffe und der als bevorzugt gekennzeichneten Einsatzmengen bzw. Einsatzmengenverhältnisse (*vide supra*) sind natürlich auch im Rahmen aller Aersosolschaum-Ausführungsform(en) bevorzugt.

Die erfindungsgemäßen Mittel und Produkte, die die erfindungsgemäßen Mittel enthalten, insbesondere Aerosolhaarsprays und Aerosolhaarschäume, zeichnen sich insbesondere dadurch aus, dass sie behandeltem Haar einen sehr natürlichen Glanz und starken Halt verleihen. Ein zweiter Gegenstand der Erfindung ist die Verwendung eines Mittels des ersten Erfindungsgegenstandes zur Erzeugung von Glanz auf keratinhaltigen Fasern, insbesondere menschlichen Haaren.

Die erfindungsgemäßen Produkte, die diese Mittel enthalten, insbesondere Aerosolhaarsprays, zeichnen sich insbesondere dadurch aus, dass sie behandeltem Haar einen sehr starken, dauerhaften Frisurenhalt verleihen, obgleich das Haar flexibel bleibt. Ein dritter Gegenstand der Erfindung ist daher die Verwendung eines Mittels des ersten Erfindungsgegenstandes zur temporären Verformung keratinhaltiger Fasern, insbesondere menschlicher Haare.

Ein vierter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Spray auf die keratinhaltigen Fasern appliziert wird.
Dabei ist es erfindungsgemäß bevorzugt, dass die keratinhaltigen Fasern in Form gebracht werden und diese Form durch das Mittel des ersten Erfindungsgegenstandes fixiert wird.

Ferner ist es bevorzugt, wenn nach der Applikation des erfindungsgemäßen Mittels das erfindungsgemäße Mittel aus den keratinhaltigen Fasern verbleibt, d.h. nicht wieder ausgespült wird.

Als erfindungsgemäß bevorzugt gelten die zuvor genannten Abgabevorrichtungen bzw. Aerosolprodukte (*vide supra*).

Ein fünfter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand zu einem Schaum verschäumt wird und der resultierende Schaum auf die keratinhaltigen Fasern appliziert wird.
Dabei ist es erfindungsgemäß bevorzugt, dass die keratinhaltigen Fasern in Form gebracht werden und diese Form durch das Mittel des ersten Erfindungsgegenstandes fixiert wird.

Ferner ist es bevorzugt, wenn nach der Applikation des erfindungsgemäßen Mittels das erfindungsgemäße Mittel aus den keratinhaltigen Fasern verbleibt, d.h. nicht wieder ausgespült wird.

Als erfindungsgemäß bevorzugt gelten die zuvor genannten Abgabevorrichtungen (*vide supra*).

### 1.0 Beispielrezepturen

Folgende Rezepturen wurden durch Vermischen der angegebenen Rohstoffe bereitgestellt und in eine Aerosoldose mit einem Ventil der Fa. Coster, Typ KE und einer Wirbeldüse der Fa. Coster "Standard micromist insert", Typ V06 abgefüllt. Die Aerosoldosen wurden mit dem Ventil verschlossen und abschließend das entsprechende Treibmittel (hier Dimethylether) zugefügt. Die folgenden Mengenangaben verstehen sich - soweit nichts anderes vermerkt ist - in Gewichtsprozent.

**Tabelle 1: Rezepturen & Vergleichsrezepturen**

| Rohstoffe | E1 | E2 | E3 | E4 | V1 | V2 | V3 | V4 |
|---|---|---|---|---|---|---|---|---|
| Ethanol | <-------------------- ad 100 --------------------> | | | | | | | |
| Wasser | 6,0 | 6,0 | 6,0 | 6,0 | 6,0 | 6,0 | 6,0 | 6,0 |
| Styrene / Acrylates Copolymer | 2,2* | 2,2* | 2,2* | 2,2* | 4,4* | - | - | - |
| Amphomer ¹ | - | - | - | - | - | 4,4* | - | 2,2* |
| Wacker Belsil^{®} P101 ² | 2,2* | - | - | - | - | - | - | - |
| Polymer 1 ³ | - | 2,2^{*} | - | - | - | - | - | - |
| Polymer 2 ⁴ | - | - | 2,2* | - | - | - | - | - |
| Polymer 3 ⁵ | - | - | - | 2,2* | - | - | 4,4* | 2,2* |
| PEG-40 Hydrogenated Castor Oil | 0,2 | 0,2 | 0,2 | 0,2 | 0,2 | 0,2 | 0,2 | 0,2 |
| Parfum | 0,1 | 0,1 | 0,1 | 0,1 | 0,1 | 0,1 | 0,1 | 0,1 |
| Dimethylether | 54,00 | 54,00 | 54,00 | 54,00 | 54,00 | 54,00 | 54,00 | 54,00 |

| | | | | | | | | |
|---|---|---|---|---|---|---|---|---|
| ^{*} Aktivsubstanz an Polymer ¹ INCI-Bezeichnung: Octylacrylamide/Acrylates/Butylaminoethyl Methacrylate Copolymer (National Starch) ² vernetztes Copolymer aus Crotonsäure, Vinyl-(C₈ bis C₁₂)-Isoalkylestern und alpha, omega-Vinyldimethicone (Säurezahl [mg KOH/g Polymer]: 28-33) (Wacker Chemie AG) ³ vernetztes Copolymer aus Crotonsäure, Vinyl-(C₈ bis C₁₂)-Isoalkylestern und alpha, omega-Vinyldimethicone (Säurezahl [mg KOH/g Polymer]: 63) ⁴ vernetztes Copolymer aus Crotonsäure, Vinyl-(C₈ bis C₁₂)-Isoalkylestern und alpha, omega-Vinyldimethicone (Säurezahl [mg KOH/g Polymer]: 50 ⁵ vernetztes Copolymer aus Crotonsäure, Vinyl-(C₈ bis C₁₂)-Isoalkylestern und alpha, omega-Vinyldimethicone (Säurezahl [mg KOH/g Polymer]: 32) | | | | | | | | |

Nach Applikation der Mittel E1 bis E4 auf je eine Haarsträhne bewirkten diese erfindungsgemäßen Mittel einen hervorragenden, Frisurenhalt. Das Haar erhielt einen natürlichen Glanz und fühlte sich geschmeidig an. Die Vergleichsrezepturen V1 bis V4 erreichten nach Applikation auf je eine Haarsträhne ein schlechteres Haargefühl, sowie geringere Glanzparameter bzw. Frisurenhaltparameter.

### 2.0 Bestimmung des Halts

### 2.1 Drei-Punkt-Biege-Test

Die Haarsträhnen (Kerling Internationale Haarfabrik GmbH, Artikel Nummer 826500 Klebetresse dicht, Typ: European natural, Farbe: 6/0, freie Haarlänge 130 mm, Breite 20 mm, Gewicht 1.8 g, an einem Ende verklebt) wurden in 1 I auf 50°C temperiertes Wasser, enthaltend 4 mL einer 25 Gew.-%-igen wässrigen Natriumlaurethsulfat-Lösung, gegeben und unter Anwendung von Ultraschall für 15 Minuten gewaschen. Die Strähnen wurden gründlich mit lauwarmen Wasser gespült und anschließend gekämmt. Das Wasser wurde vorsichtig aus dem Haar mittels Daumen und Zeigefinger gedrückt. Dieser Vorgung wurde insgesamt pro Strähne drei Male durchgeführt.

Die Haarsträhnen wurden an einem Ende an einem Rahmen aufgehängt. An das andere Ende der Haarsträhne wurde ein Gewicht befestigt, so dass das Haar gerade herab hing. Die obere Hälfte und die untere Hälfte einer Strähnenseite wurden mit einem der Testhaarsprays jeweils aus einer Distanz von 30 cm für eine Dauer von 10 Sekunden besprüht. Der Rahmen wurde um 180° gedreht und die gegenüberliegende Seite der zuvor besprühnten Seite wurde ebenfalls wie zuvor beschrieben besprüht. Die Haarsträhnen wurden über Nacht bei 296 K und einer relativen Luftfeuchtigkeit von 50% getrocknet. Danach wurden die Strähnen vorsichtig aus dem Rahmen entfernt.

Je Testhaarspray wurden 5 Haarsträhnen präpariert und mit dem Messgerät "Lloyd Intruments LF Plus" jeweils die maximal aufgewendete Kraft bis zum Bruch des Polymerfilms bestimmt. Dabei befand sich zu Messbeginn der Belastungsstempel des Messgeräts 40 mm von der Teststrähne entfernt und wurde mit einer Geschwindigkeit von 500 mm/Minute auf die Strähne zubewegt. Es wurden die Mittelwerte der Messwerte berechnet und diese auf statistische Signifikanz mittels Student t-Test geprüft. Alle Werte waren statistisch signifikant. Es ergaben sich folgende Ergebnisse:

**Tabelle 2: Messergebnisse & Erwartungswert**

| Haarspray (vgl. Tabelle 1) | Fₘₐₓ / N | Erwartungswert Fₘₐₓ /N | Differenz zum Erwartungswert |
|---|---|---|---|
| V1 | 7.1 | - | - |
| V2 | 6.3 | - | - |
| V3 | 4.2 | - | - |
| V4 | 5.8 | 5.3 | + 0.5 |
| E4 | 6.7 | 5.7 | + 1,0 |

Der Haltegrad der erfindungsgemäßen Mischung E4 ist höher, als der berechnete Erwartungswert der Mischung. Ferner senkt sich der Haltegrad von E4 im Vergleich zum Haltegrad einer Mischung aus Amphomer und dem vernetzen anionischen Copolymer (V4) signifikant weniger ab. Die erfindungsgemäße Polymerkombination erzielt demnach neben hervorragenden Parametern bezüglich Glanz und Haargefühl ein optimal haltbares Styling.

## Patentansprüche

1. Mittel zur Behandlung keratinhaltiger Fasern, insbesondere menschlichem Haar, enthaltend in einem kosmetisch akzeptablen Träger
- mindestens ein erstes festigendes anionisches Copolymer, umfassend mindestens eine Struktureinheit der Formel (I) und mindestens eine Struktureinheit der Formel (II) und mindestens eine Struktureinheit der Formel (III) 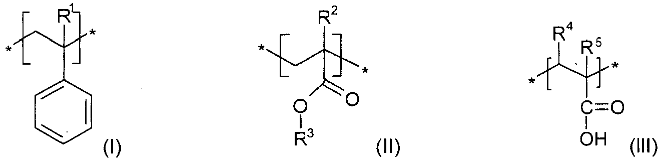 worin
R¹ steht für ein Wasserstoffatom oder eine Methylgruppe,
R² steht für ein Wasserstoffatom oder eine Methylgruppe
R³ steht für eine (C₁ bis C₂₀)-Alkylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
R⁴ und R⁵ stehen für ein Wasserstoffatom oder einer der beiden Reste für ein Wasserstoffatom und der andere für eine Methylgruppe
und
- mindestens ein zweites vernetztes anionisches Copolymer umfassend mindestens eine vernetzende Polysiloxan-Struktureinheit der Formel (PS) und mindestens eine Struktureinheit der Formel (IV) und mindestens eine Struktureinheit der Formel (V) 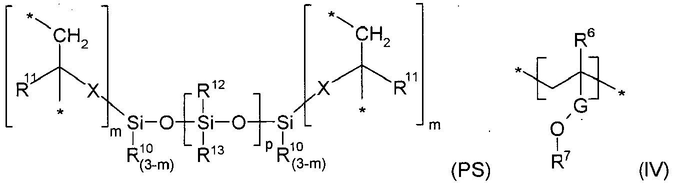 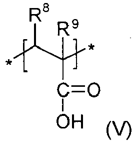 worin
R⁶ ein Wasserstoffatom oder eine Methylgruppe bedeutet,
R⁷ steht für eine lineare oder verzweigte (C₁ bis C₂₀)-Alkylgruppe, lineare oder verzweigte (C₂ bis C₂₀)-Acylgruppe, eine (C₂ bis C₆)-Hydroxyalkylgruppe oder eine Gruppe *-(CH₂CH₂O)ₙ-(C₁ bis C₂₀)-Alkyl mit n = 1 bis 30,
G eine direkte Bindung oder eine Carbonylgruppe bedeutet,
R⁸ und R⁹ stehen für ein Wasserstoffatom oder einer der beiden Reste für ein Wasserstoffatom und der andere für eine Methylgruppe,
R¹¹ steht für ein Wasserstoffatom oder eine Methylgruppe,
R¹⁰ R¹², R¹³ stehen unabhängig voneinander für eine monovalente, Si-C-gebundene, gegebenenfalls substituierte Kohlenwasserstoffgruppe mit 1 bis 18 Kohlenstoffatomen (insbesondere Methyl oder Phenyl) oder eine (C₁ bis C₁₈)-Alkoxygruppe (insbesondere Methoxy oder Ethoxy),
m für eine ganze Zahl von 1 bis 3 steht
p für eine ganze Zahl von 10 bis 1000 steht,
X steht für eine direkte Bindung oder ein beliebiges Molekülfragment.

2. Mittel nach Anspruch 1, **dadurch gekennzeichnet, dass** das erste festigende anionische Copolymere, mindestens eine Struktureinheit der Formel (I) und mindestens eine Struktureinheit der Formel (IIa) und mindestens eine Struktureinheit der Formel (IIb) und mindestens eine Struktureinheit der Formel (III) umfasst 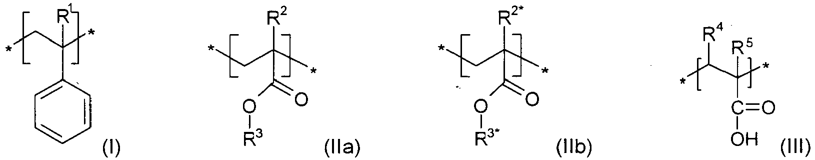 worin
R¹ steht für ein Wasserstoffatom oder eine Methylgruppe,
R² steht für ein Wasserstoffatom oder eine Methylgruppe
R³ steht für eine (C₁ bis C₂₀)-Alkylgruppe oder eine Gruppe *-(CH₂CH₂O)n-(C₁ bis C₂₀)-Alkyl mit n = 1 bis 30,
R^{2*} steht für ein Wasserstoffatom oder eine Methylgruppe
R^{3*} steht für eine (C₂ bis C₆)-Hydroxyalkylgruppe,
R⁴ und R⁵ stehen für ein Wasserstoffatom oder einer der beiden Reste für ein Wasserstoffatom und der andere für eine Methylgruppe.

3. Mittel nach einem der vorangehenden Ansprüche, **dadurch gekennzeichnet, dass** der Rest R¹ gemäß Formel (I) für ein Wasserstoffatom steht.

4. Mittel nach einem der vorangehenden Ansprüche, **dadurch gekennzeichnet, dass** R³ gemäß Formel (II) (bzw. R³ gemäß Formel (IIa) und R^{3*} gemäß Formel (IIb) unabhängig voneinander) stehen für eine Methylgruppe, Ethylgruppe, 2-Hydroxyethylgruppe, 2,3-Dihydroxypropylgruppe, n-Propylgruppe, Isopropylgruppe, n-Butylgruppe, sec-Butylgruppe oder tert-Butylgruppe.

5. Mittel nach einem der Ansprüche 2 bis 4, **dadurch gekennzeichnet, dass** R³ gemäß Formel (IIa) steht für eine Methylgruppe, Ethylgruppe, n-Propylgruppe, Isopropylgruppe, n-Butylgruppe, sec-Butylgruppe oder tert-Butylgruppe.

6. Mittel nach einem der Ansprüche 2 bis 5, **dadurch gekennzeichnet, dass** R^{3*} gemäß Formel (IIb) steht für eine 2-Hydroxyethylgruppe oder 2,3-Dihydroxypropylgruppe.

7. Mittel nach einem der Ansprüche 1 bis 6, **dadurch gekennzeichnet, dass** das erste festigende anionische Copolymer bevorzugt in einer Menge von 0.01 bis 29.99 Gew.-%, besonders bevorzugt von 0,1 bis 14,9 Gew.-%, ganz besonders bevorzugt von 0,1 bis 9,5 Gew.-%, und am bevorzugtesten von 0,2 bis 5,0 Gew.-%, jeweils bezogen auf das Gewicht des gesamten anwendungsbereiten Mittels, enthalten ist.

8. Mittel nach einem der Ansprüche 1 bis 7, **dadurch gekennzeichnet, dass** das zweite vernetzte anionische Copolymer als vernetzende Struktureinheit der Formel (PS) mindestens eine vernetzende Struktureinheit der Formel (PS-1) umfasst 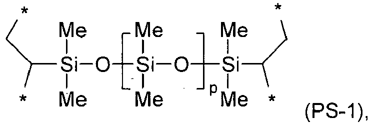 worin p für eine ganze Zahl von 10 bis 1000 steht.

9. Mittel nach einem der Ansprüche 1 bis 8, **dadurch gekennzeichnet, dass** das zweite vernetzte anionische Copolymer bevorzugt in einer Menge von 0.01 bis 29.99 Gew.-%, besonders bevorzugt von 0,1 bis 14,9 Gew.-%, ganz besonders bevorzugt von 0,1 bis 9,5 Gew.-%, und am bevorzugtesten von 0,2 bis 5,0 Gew.-%, jeweils bezogen auf das Gewicht des gesamten anwendungsbereiten Mittels, enthalten ist.

10. Mittel nach einem der Ansprüche 1 bis 9, **dadurch gekennzeichnet, dass** es zusätzlich mindestens ein Tensid enthält.

11. Verwendung eines Mittels gemäß einem der Ansprüche 1 bis 10 zur Erzeugung von Glanz auf keratinhaltigen Fasern, insbesondere menschlichen Haaren.

12. Verwendung eines Mittels gemäß einem der Ansprüche 1 bis 10 zur temporären Verformung keratinhaltiger Fasern, insbesondere menschlicher Haare.

13. Verfahren zur Behandlung keratinhaltiger Fasern, insbesondere menschlicher Haare, worin unter Einsatz einer Abgabevorrichtung ein Mittel gemäß einem der Ansprüche 1 bis 10 als Spray auf die keratinhaltigen Fasern appliziert wird.

14. Verfahren zur Behandlung keratinhaltiger Fasern, insbesondere menschlicher Haare, worin unter Einsatz einer Abgabevorrichtung ein Mittel gemäß einem der Ansprüche 1 bis 10 zu einem Schaum verschäumt wird und der resultierende Schaum auf die keratinhaltigen Fasern appliziert wird.

## Claims

1. An agent for treating keratin-containing fibers, in particular human hair, containing
- at least one first fixative anionic copolymer comprising at least one structural unit of the formula (I) and at least one structural unit of the formula (II) and at least one structural unit of the formula (III) 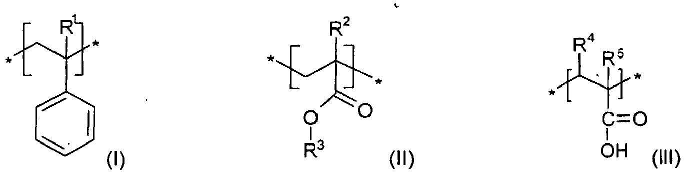 where
R¹ represents a hydrogen atom or a methyl group,
R² represents a hydrogen atom or a methyl group,
R³ represents a (C₁ to C₂₀) alkyl group, a (C₂ to C₆) hydroxyalkyl group or a *-(CH₂CH₂O)ₙ-(C₁ to C₂₀) alkyl group, where n = 1 to 30,
R⁴ and R⁵ represent a hydrogen atom, or one of the two functional groups represents a hydrogen atom and the other represents a methyl group,
and
- at least one second crosslinked anionic copolymer comprising at least one crosslinking polysiloxane structural unit of the formula (PS) and at least one structural unit of the formula (IV) and at least one structural unit of the formula (V) 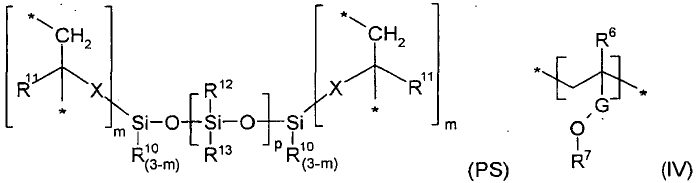 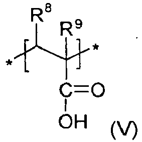 in a cosmetically acceptable carrier,
where
R⁶ is a hydrogen atom or a methyl group,
R⁷ represents a linear or branched (C₁ to C₂₀) alkyl group, a linear or branched (C₂ to C₂₀) acyl group, a (C₂ to C₆) hydroxyalkyl group or a *-(CH₂CH₂O)ₙ-(C₁ to C₂₀) alkyl group, where n = 1 to 30,
G is a direct bond or a carbonyl group,
R⁸ and R⁹ represent a hydrogen atom, or one of the two functional groups represents a hydrogen atom and the other represents a methyl group,
R¹¹ represents a hydrogen atom or a methyl group,
R¹⁰, R¹² and R¹³ represent, independently of one another, a monovalent, Si-C-bonded, optionally substituted hydrocarbon group having from 1 to 18 carbon atoms (in particular methyl or phenyl) or a (C₁ to C₁₈) alkoxy group (in particular methoxy or ethoxy),
m represents an integer of from 1 to 3,
p represents an integer of from 10 to 1000, and
X represents a direct bond or any molecular fragment.

2. The agent according to claim 1, **characterized in that** the first fixative anionic copolymer comprises at least one structural unit of the formula (I) and at least one structural unit of the formula (IIa) and at least one structural unit of the formula (IIb) and at least one structural unit of the formula (III) 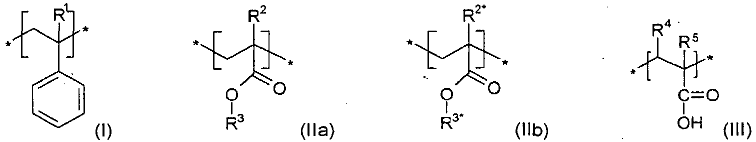 where
R¹ represents a hydrogen atom or a methyl group,
R² represents a hydrogen atom or a methyl group,
R³ represents a (C1 to C₂₀) alkyl group or a *-(CH₂CH₂O)ₙ-(C₁ to C₂₀) alkyl group, where n = 1 to 30,
R^{2*} represents a hydrogen atom or a methyl group,
R^{3*} represents a (C₂ to C₆) hydroxyalkyl group, and
R⁴ and R⁵ represent a hydrogen atom, or one of the two functional groups represents a hydrogen atom and the other represents a methyl group.

3. The agent according to one of the preceding claims, **characterized in that** the functional group R1 according to formula (I) represents a hydrogen atom.

4. The agent according to one of the preceding claims, **characterized in that** R3 according to formula (II) (or R3 according to formula (IIa) and R3* according to formula (IIb), independently of one another) represent a methyl group, an ethyl group, a 2-hydroxyethyl group, a 2,3-dihydroxypropyl group, an n-propyl group, an isopropyl group, an n-butyl group, a sec-butyl group or a tert-butyl group.

5. The agent according to one of claims 2 to 4, **characterized in that** R3 according to formula (IIa) represents a methyl group, an ethyl group, an n-propyl group, an isopropyl group, an n-butyl group, a sec-butyl group or a tert-butyl group.

6. The agent according to one of claims 2 to 5, **characterized in that** R3* according to formula (IIb) represents a 2-hydroxyethyl group or a 2,3-dihydroxypropyl group.

7. The agent according to one of claims 1 to 6, **characterized in that** said agent preferably contains the first fixative anionic copolymer in an amount of from 0.01 to 29.99 wt.%, more preferably from 0.1 to 14.9 wt.%, particularly preferably from 0.1 to 9.5 wt.% and most preferably from 0.2 to 5.0 wt.%, based on the weight of the overall ready-to-use agent in each case.

8. The agent according to one of claims 1 to 7, **characterized in that** the second crosslinked anionic copolymer comprises, as the crosslinking structural unit of the formula (PS), at least one crosslinking structural unit of the formula (PS-1), 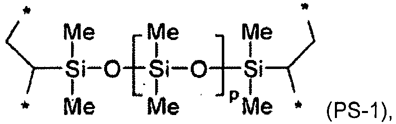 where p represents an integer of from 10 to 1000.

9. The agent according to one of claims 1 to 8, **characterized in that** said agent preferably contains the second crosslinked anionic copolymer in an amount of from 0.01 to 29.99 wt.%, more preferably from 0.1 to 14.9 wt.%, particularly preferably from 0.1 to 9.5 wt.% and most preferably from 0.2 to 5.0 wt.%, based on the weight of the overall ready-to-use agent in each case.

10. The agent according to one of claims 1 to 9, **characterized in that** said agent also contains at least one surfactant.

11. The use of an agent according to one of claims 1 to 10 for creating a shine on keratin-containing fibers, in particular human hair.

12. The use of an agent according to one of claims 1 to 10 for temporarily styling keratin-containing fibers, in particular human hair.

13. A method for treating keratin-containing fibers, in particular human hair, wherein a dispensing device is used to apply an agent according to one of claims 1 to 10 to the keratin-containing fibers in the form of a spray.

14. A method for treating keratin-containing fibers, in particular human hair, wherein a dispensing device is used to foam an agent according to one of claims 1 to 10 to form a mousse and to apply the resultant mousse to the keratin-containing fibers.

## Revendications

1. Agent de traitement de fibres kératiniques, notamment du cheveu humain, comprenant dans un support cosmétiquement acceptable
- au moins un premier copolymère anionique fixant, comprenant au moins un motif structural de formule (I) et au moins un motif structural de formule (II) et au moins un motif structural de formule (III) 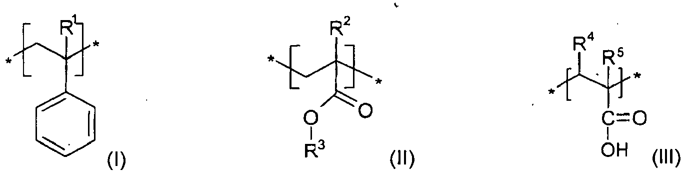 dans lesquelles
R¹ représente un atome d'hydrogène ou un groupe méthyle,
R² représente un atome d'hydrogène ou un groupe méthyle
R³ représente un groupe alkyle (en C₁ à C₂₀), un groupe hydroxyalkyle (en C₂ à C₆) ou un groupe *-(CH₂CH₂O)ₙ-alkyle-(en C₁ à C₂₀) sachant que n = 1 à 30,
R⁴ et R⁵ représentent un atome d'hydrogène ou un des deux résidus représente un atome d'hydrogène et l'autre, un groupe méthyle
et
- au moins un deuxième copolymère anionique réticulé comprenant au moins un motif structural de polysiloxane réticulant de formule (PS) et au moins un motif structural de formule (IV) et au moins un motif structural de formule (V) 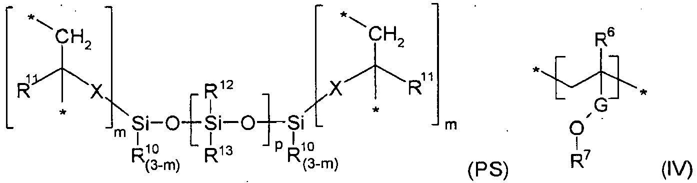 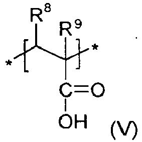 dans lesquelles
R⁶ désigne un atome d'hydrogène ou un groupe méthyle,
R⁷ représente un groupe alkyle linéaire ou ramifié (en C₁ à C₂₀), un groupe acyle linéaire ou ramifié (en C₂ à C₂₀), un groupe hydroxyalkyle (en C₂ à C₆) ou un groupe *-(CH₂CH₂O)ₙ-alkyle-(en C₁ à C₂₀) sachant que n = 1 à 30,
G désigne une liaison directe ou un groupe carbonyle,
R⁸ et R⁹ représentent un atome d'hydrogène ou un des deux résidus représente un atome d'hydrogène et l'autre, un groupe méthyle,
R¹¹ représente un atome d'hydrogène ou un groupe méthyle,
R¹⁰, R¹², R¹³ représentent, indépendamment les uns des autres, un groupe hydrocarbure monovalent, à liaison Si-C, éventuellement substitué, comportant 1 à 18 atomes de carbone (en particulier méthyle ou phényle) ou un groupe alkoxy (en C₁ à C₁₈) (en particulier méthoxy ou éthoxy),
m représente un nombre entier allant de 1 à 3
p représente un nombre entier allant de 10 à 1000,
X représente une liaison directe ou un quelconque fragment moléculaire.

2. Agent selon la revendication 1, **caractérisé en ce que** le premier copolymère anionique fixant comprend au moins un motif structural de formule (I) et au moins un motif structural de formule (IIa) et au moins un motif structural de formule (IIb) et au moins un motif structural de formule (III) 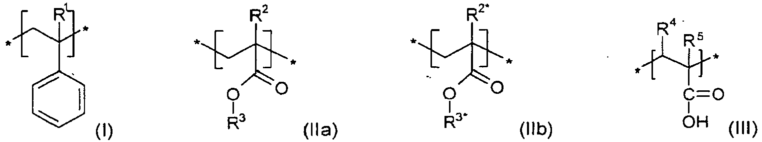 dans lesquelles
R¹ représente un atome d'hydrogène ou un groupe méthyle,
R² représente un atome d'hydrogène ou un groupe méthyle
R³ représente un groupe alkyle (en C₁ à C₂₀) ou un groupe *-(CH₂CH₂O)ₙ-alkyle-(en C₁ à C₂₀) sachant que n = 1 à 30
R^{2*} représente un atome d'hydrogène ou un groupe méthyle
R^{3*} représente un groupe hydroxyalkyle (en C₂ à ₆),
R⁴ et R⁵ représentent un atome de carbone ou un des deux résidus représente un atome d'hydrogène et l'autre, un groupe méthyle.

3. Agent selon l'une quelconque des revendications précédentes **caractérisé en ce que** le résidu R1 selon la formule (I) représente un atome de carbone.

4. Agent selon l'une quelconque des revendications précédentes **caractérisé en ce que** R3 selon la formule (II) (ou R3 selon la formule (IIa) et R3* selon la formule (IIb) indépendamment l'un de l'autre) représentent un groupe méthyle, un groupe éthyle, un groupe 2-hydroxyéthyle, un groupe 2,3-dihydroxypropyle, un groupe n-propyle, un groupe isopropyle, un groupe n-butyle, un groupe sec-butyle ou un groupe tert-butyle.

5. Agent selon une des revendications 2 à 4, **caractérisé en ce que** R3 selon la formule (IIa) représente un groupe méthyle, un groupe éthyle, un groupe n-propyle, un groupe isopropyle, un groupe n-butyle, un groupe sec-butyle ou un groupe tert-butyle.

6. Agent selon une des revendications 2 à 5, **caractérisé en ce que** R3* selon la formule (IIb) représente un groupe 2-hydroxyéthyle ou un groupe 2,3-dihydroxypropyle.

7. Agent selon une des revendications 1 à 6, **caractérisé en ce que** le premier copolymère anionique fixant est compris de préférence dans une quantité allant de 0,01 à 29,99 % en poids, de manière davantage préférée de 0,1 à 14,9 % en poids, de manière particulièrement préférée de 0,1 à 9,5 % en poids et de manière préférée entre toutes de 0,2 à 5,0 % en poids, par rapport au poids total de l'agent prêt à l'emploi respectivement.

8. Agent selon une des revendications 1 à 7, **caractérisé en ce que** le deuxième copolymère anionique réticulé comprend, comme motif structural réticulant de formule (PS), au moins un motif structural réticulant de formule (PS-1) 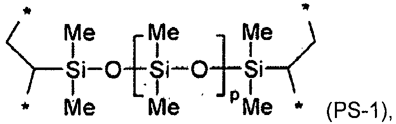 dans laquelle p représente un nombre entier allant de 10 à 1000.

9. Agent selon une des revendications 1 à 8, **caractérisé en ce que** le deuxième copolymère anionique réticulé est compris de préférence dans une quantité allant de 0,01 à 29,99 % en poids, de manière davantage préférée de 0,1 à 14,9 % en poids, de manière particulièrement préférée de 0,1 à 9,5 % en poids et de manière préférée entre toutes de 0,2 à 5,0 % en poids, par rapport au poids total de l'agent prêt à l'emploi respectivement.

10. Agent selon une des revendications 1 à 9, **caractérisé en ce qu'**il comprend, en plus, au moins un tensioactif.

11. Utilisation d'un agent selon une des revendications 1 à 10 pour la production d'un éclat sur des fibres kératiniques, notamment des cheveux humains.

12. Utilisation d'un agent selon une des revendications 1 à 10 pour la mise en forme temporaire de fibres kératiniques, notamment de cheveux humains.

13. Procédé de traitement de fibres kératiniques, notamment de cheveux humains, dans lequel, en utilisant un dispositif de distribution, un agent selon une des revendications 1 à 10 est appliqué sous forme de spray sur les fibres kératiniques.

14. Procédé de traitement de fibres kératiniques, notamment de cheveux humains, dans lequel, en utilisant un dispositif de distribution, un agent selon une des revendications 1 à 10 est expansé en mousse et la mousse en résultant est appliquée sur les fibres kératiniques.
